# EUROPEAN PATENT SPECIFICATION

(11) **EP 0 501 692 B1**
(45) Date of publication and mention of the grant of the patent: **02.08.2000**
(21) Application number: 92301466.6
(22) Date of filing: 21.02.1992
(51) Int. Cl.: C12N 15/29, C12P 21/02, C12N 15/70, C07K 4/00

(54) **Fermentation processes for the production of peptides by a host**
Fermentationsverfahren zur Herstellung von Peptiden durch einen Wirt
Procédé de fermentation pour la production de peptides par une hôte

(30) Priority: 26.02.1991 GB 9103925; 26.02.1991 GB 9103926; 26.02.1991 GB 9104016
(43) Date of publication of application: 02.09.1992
(73) Proprietor: ZENECA LIMITED, London W1Y 6LN (GB)
(72) Inventor: Kara, Bhuphendra Vallabh, Macclesfield, Cheshire, GB-SK10 4TG (GB); Hockney, Robert Craig, Macclesfield, Cheshire, GB-SK10 4TG (GB); Fitton, John Edward, Macclesfield, Cheshire, GB-SK10 4TG (GB)
(74) Representative: Phillips, Neil Godfrey Alasdair

(56) References cited:
- EP-A- 0 144 064
- EP-A- 0 237 676
- BIO/TECHNOLOGY vol. 7, no. 11, November 1989, NATURE AMERICA, INC., NEW YORK, US pages 1141 - 1149 C.H. SCHEIN 'Production of soluble recombinant proteins in bacteria'
- J. BIOL. CHEM. vol. 263, no. 10, 5 April 1988, AM. SOC. MOL. BIOL., INC. US; pages 4837 - 4843 M. PIATAK ET AL. 'Expression of soluble and fully functional Ricin A chain in Escherichia coli is temperature-sensitive'
- FEBS LETT. vol. 216, no. 1, May 1987, ELSEVIER PUBLISHERS, AMSTERDAM, NL; pages 73 - 78 M. O'HARE ET AL. 'Expression of ricin A chain in Escherichia coli'
- J. INDUSTRIAL MICROBIOLOGY vol. 2, no. 3, August 1987, ELSEVIER PRESS, AMSTERDAM, NL; pages 181 - 187 L.B. TSAI ET AL. 'The effect of organic nitrogen and glucose on the production of recombinant human insulin-like growth factor in high cell density Escherichia coli fermentations'

## Description

This invention relates to the field of biotechnology and is particularly concerned with processes for preparing soluble recombinant molecules.

Generally when recombinant proteins are prepared in bacterial hosts, such as E.coli, the fermentation is carried out under conditions which favour growth, that is at a temperature of about 37°C and a pH of about 6.8.

The fermentation process is usually carried out in a medium which contains those nutrients which fulfill the cellular growth requirements of the host. Typically the growth medium includes sources of carbon and nitrogen for synthesis of cellular components and energy, ions such as sulphate, phosphate, magnesium, calcium, iron and various trace elements. Yeast extract is also often present as a component of the growth medium. For example, Luria Broth contains about 0.5% yeast extract in addition to tryptone and sodium chloride. Recently it has been reported (X Li, J W Robbins and K B Taylor: Journal of Industrial Microbiology, 5, 85-94, 1990) that when Luria Broth is enriched with yeast extract so that it contains 1 to 3 % yeast extract biomass and β-galactosidase expression is increased. It has also been reported (L.B. Tsai et al, Journal of Industrial Microbiology, 2, 181-187, 1987) that recombinant human insulin-like growth factor can be prepared as inclusion bodies by feeding of yeast extract and glucose into the fermentation broth in a fed-batch process.

US 4,894,334 describes a process for the preparation of ricin A in which the cultivation is carried out at 37°C and at a pH of 6.8, and a 2% solution of casamino acid is added when the turbidity of the growth medium is about OD₆₈₀ of 10.

When many polypeptides are prepared by recombinant DNA technology in E.coli the polypeptide is obtained in an insoluble form, for example as insoluble aggregates or inclusion bodies. If this material is to be used, it has to be made soluble to permit renaturation into biologically active form. This solubilisation step generally requires the use of chemicals, such as detergents or chaotropic agents, which are tedious and expensive to use and can lead to chemical modification of the protein.

The production of insoluble material tends to occur when recombinant ricin A is prepared. For example, it is reported in EP 237,676 that expression of ricin A in E.coli at 37°C, pH 6.8 leads to insoluble material which has to be solubilised by treatment with a mixture of urea and SDS. EP 237,676 reports that soluble ricin A is produced when the coding sequence for ricin A is placed in direct reading frame with the DNA encoding the leader sequence of the alkaline phosphatase structural gene (phoA) of E.coli K12.

Ricin and ricin-type molecules, such as abrin, are known compounds which are produced by plant cells, and which possess cytotoxic properties. Toxins of this type consist of two polypeptide chains which are linked via a disulphide bridge. One of the polypeptide chains (the "A chain") is primarily responsible for the cytotoxic properties of the toxin molecule; whilst the other polypeptide chain (the "B chain") enables the toxin molecule to bind to cell surfaces.

The toxicity of ricin is dependent upon three distinguishable events:-
(i) binding of the ricin molecule to the cell surface through interaction of galactose binding sites on the B chain with glycoproteins or glycolipids exposed at the cell surface;
(ii) penetration of at least the A chain into the cytosol of the cell; and
(iii) enzymic cleavage of RNA in the 60S sub-unit of the ribosome leading to inhibition of protein synthesis and ultimately cell death.

It is also believed that the B chain plays an important secondary function, apart from its primary function of binding the ricin molecule to the cell surface, in that it facilitates uptake of ricin into the cell. Thus separated A and B chains are essentially non-toxic since the B chain is not cytotoxic, and the A chain lacks the ability to bind to cell surfaces and penetrate into the cytosol of the cell in the absence of the B chain.

It has already been suggested that the toxicity of the A chain of ricin might be useful in anti-tumour therapy if the indiscriminately-binding B chain could be replaced by a different carrier which has the ability to bind to tumour cells in preference to normal cells. Thus, it has been proposed that an immunotoxin which comprises ricin A and a tumour-specific antibody may be of use in anti-tumour therapy.

The preparation of the A chain of ricin from natural sources, such as from the seeds of Ricinus communis, is difficult. In particular it is difficult to purify ricin A, that is to separate the A chain from the B chain.

Although it is possible to prepare polypeptides, such as ricin A, by recombinant DNA technology, there is still a need for improved processes for the preparation of soluble polypeptides. In particular there is a need for improved processes for the preparation of soluble ricin A.

According to the present invention there is provided a process for preparing a polypeptide, which process comprises cultivating a host capable of expressing said polypeptide in a growth medium and cultivating said host for an initial period at a first pH value which favours growth of the host so that polypeptide is produced; adjusting the pH to a second value
which favours accumulation of soluble polypeptide and cultivating the host for a further period at said second pH value;
and optionally reducing the temperature of the growth medium during the terminal portion of the cultivation;
such that soluble polypeptide may be obtained.

In particular the process of the present invention may be used to prepare polypeptides which have a tendency to be produced, at least in part, in an insoluble form.

Particular examples of polypeptides which may be produced using the present invention include, for example, ricin A or an analogue thereof.

In one embodiment of the present invention there is provided a process for preparing a polypeptide, which process comprises cultivating a host capable of expressing said polypeptide and adjusting the pH during the process such that soluble polypeptide may be obtained. In particular there is provided a method of preparing a polypeptide, which method comprises cultivating a host which is capable of expressing said polypeptide in a growth medium for an initial period at a first pH value which favours growth of the host; adjusting the pH to a second value which favours accumulation of soluble polypeptide and cultivating the host for a further period at said second pH value.

In processes where the temperature is reduced, the process will in general comprise cultivating the host cells at a temperature which favours growth of the host cells and generation of soluble polypeptide, cooling growth medium (and hence the host) during the terminal portion of the cultivation, and harvesting the host during said terminal portion.

The temperature is preferably one which favours maintenance of the polypeptide in a soluble form.

Suitable temperatures which favour growth of bacterial cells, such as E . coli, are those from about 25°C to about 39°C (for example 37 or 38°C), with the optimum temperature being about 37°C. The temperature should preferably be one which leads to the generation of soluble polypeptide and so, in general, the temperature will be below 40°C. In general, it is preferred that when the temperature is reduced, the host cells are cooled to a temperature below about 25°C, for example to a temperature in the range from about 10°C (or below) to about 25°C.

It is preferred that cooling is effected at the point where the accumulation of soluble polypeptide (such as ricin A) within the cells is high and most preferably at or near the maximum specific activity.

It is generally preferred that when the pH is adjusted, the host which is capable of expressing the polypeptide is cultivated in a growth medium for an initial period at a first pH value which favours growth of the host, and the pH is adjusted to a second value and the host cultivated for a further period at this second value. The pH may be adjusted in a number of ways, for example it may be adjusted in one step or in a series of steps. The second pH may, for example, comprise a value which favours accumulation of soluble polypeptide in that it facilitates the maintenance and recovery of polypeptide in a soluble form.

Thus in a particular embodiment there is provided a process for preparing a polypeptide, which process comprises cultivating a host capable of expressing said polypeptide at a first pH value and at a temperature which favours growth of the host and generation of soluble polypeptide, adjusting the pH during the process to a second pH value, and optionally cooling the host during the terminal portion of the cultivation, and harvesting the host containing soluble polypeptide during said terminal portion.

In the case of ricin A, the pH is generally adjusted so that the said second pH value will be lower than the first pH value. For other polypeptides the said second pH value may be greater or may be lower than the first value, depending on the nature of the polypeptide.

In a particular embodiment, when the pH is adjusted the value of the pH is changed from a first value to a second value which is lower than the first value. As indicated above, the pH may be lowered in a number of ways, for example it may be lowered in one step or in a series of steps.

It is generally preferred for example, that the pH is adjusted from the first value to the second value over a short period of time, and in particular the pH may be adjusted directly from the first value to the second value.

Particular values for the pH during the initial period of the cultivation include, for example, those in the range of about 6 to 8. A preferred value for the pH during during the initial period is, for example, 6.7 or about 6.7. Values of pH which are about 6.7 include, for example, values of 6.7 +/- 0.1, and hence include a value of 6.8.

Particular values for the pH during the further period of cultivation are those in the range of about 5.5 to 8, and in particular in the range of about 6 to 8. For example, where the pH during the initial period, that is said first value, is about 6.7, a particular value for the pH during the further period is one which has a value lower than 6.7 and greater than about 5.5 (especially a value greater than, or equal to, about 6.0).

In particular the present invention provides a process for preparing ricin A or an analogue thereof. Thus, according to the present invention there is provided a method of preparing ricin A or an analogue thereof, which method comprises cultivating a host which is capable of expressing ricin A, or an analogue thereof, in a nutrient medium for an initial period at a first pH value which favours growth of the host; and cultivating the host for a further period at a pH lower than said first pH value; and optionally cooling the host during the terminal portion of the cultivation and harvesting the host during said terminal portion.

Analogues of ricin A include polypeptides which exhibit cytotoxic activity and which have a primary structure which is related to ricin A in the sense that it differs from that of ricin A by one or more amino acid alterations (deletions, additions, substitutions) which do not result in loss of cytotoxic acitivity. The preparation of hosts which are capable of expressing ricin A is described, for example in EP 145,111; WO 85/3508; and Lamb et al. Eur. J. Biochem., 1985, 148, p265-270.

As mentioned above polypeptides which possess cytotoxic properties such as ricin A may be used to prepare immunotoxins for use in treatment of tumours. Generally, such immunotoxins comprise an antibody specific for the tumour to be treated and the toxin. An example of the preparation of an immunotoxin is described in published PCT Patent Application no. WO 85/003508.

It is generally preferred, for example, that the host comprises bacterial cells such as E.coli cells. In the case of ricin A it is preferred that the host comprises an E.coli strain such as DS410.

The host is generally cultivated in a medium which contains those nutrients which fulfill the cellular growth requirements of the host. Thus the medium will, in general, include sources of carbon and nitrogen for synthesis of cellular components and energy, ions such as sulphate, phosphate, magnesium, calcium, iron and trace elements.

It is prefered that yeast extract is added to the fermentation medium during the course of the fermentation. The modes of addition of the yeast extract include those mentioned below.

The host will include a DNA sequence which codes for the polypeptide (such as ricin A or an analogue thereof) and which is under the control of appropriate control sequences such as a promoter sequence, ribosome binding site and transcription terminator sequence. Particular examples of suitable promoter sequences are the tryptophan (trp) promoter and the T7A3 promoter. Other promoters such as the lac or tac promoter may also be used. Thus, for example, the host may include a DNA sequence which codes for ricin A and which is under the control of the trp or T7A3 promoters.

In a particularly preferred embodiment the process of the present invention comprises a fermentation of the "fed-batch type".

As used herein the term a process of the "fed-batch type" refers to a fermentation process in which the batch growth medium contains a nutrient, such as carbon source, or a number of nutrients that will when exhausted limit the growth of the microorganism. When that nutrient or nutrients become exhausted a feed of that nutrient/nutrients is initiated. The point at which it becomes necessary to feed this nutrient corresponds to the onset of fed-batch conditions. The fermentation medium may be supplied with other nutrients throughout or at stages during the fermentation.

When the process comprises a fed-batch fermentation process the pH may be lowered at or before the point at which fed-batch conditions are attained, preferally before fed-batch conditions are attained.

In a preferred embodiment there is provided a fermentation process of the fed-batch type for preparing ricin A, which process comprises cultivating an E.coli host which is capable of expressing ricin A at a pH value of about 6.7 for an initial period and cultivating the host for a further period at a pH lower than said value of 6.7.

According to a preferred embodiment of the present invention there is provided a fermentation process of the fed-batch type for preparing ricin A or an analogue thereof, which process comprises cultivating a host capable of expressing ricin A, or an analogue thereof, in a growth medium for an initial period at a first pH value and at a temperature which favours growth of the host and generation of soluble ricin A or ricin A analogue; adjusting the pH before fed-batch conditions are attained to a second value which is lower than said first value; and optionally cooling the growth medium during the terminal portion of the cultivation to a temperature which favours maintanance of ricin A in a soluble form and harvesting the cells during said terminal portion.

Prior to implementation of the processes claimed in this application it was found that fermentation processes of the fed-batch type resulted in decreased yields of soluble ricin A recoverable at the end of the fermentation process. It has been suprisingly discovered that if cooling is effected before fed-batch conditions are attained then high levels of polypeptide may be maintained in soluble form , thus facilitating harvesting of soluble polypeptide.

Thus in a further embodiment of the present invention there is provided a process for preparing ricin A or an analogue thereof, which process comprises
(a) cultivating a host capable of expressing ricin A, or an analogue thereof, in a growth medium at a temperature which favours growth of the host and generation of soluble ricin A or ricin A analogue;
(b) cooling the growth medium before exhaustion of a nutrient essential for growth of the host is reached (that is fed-batch conditions are attained) to a temperature which does not favour growth of the host; and
(c) harvesting the cells before exhaustion of said nutrient is reached.

It will be appreciated that this process is similar to a fed-batch process in which harvesting takes place before the process enters the fed-batch phase. Thus the point at which exhaustion of a nutrient essential for growth of the host occurs is equivalent to the point at which fed-batch conditions are attained. Cooling is effected before nutrient exhaustion is reached, and preferably takes place, for example, just before or immediately before nutrient exhaustion is reached.

The host may be grown in a medium which is formulated to become carbon-limited at an OD₅₅₀ of about 50, and the fermentation medium containing the host is cooled when the OD₅₅₀ is about 50 or before the OD₅₅₀ reaches 50.

In particular when it is desired to reduce the temperature in a fed-batch process for the preparation of ricin A, the process may include the steps of:
(a) cultivating a E.coli host capable of expressing ricin A in a nutrient medium at a temperature of in the range from about 37°C to about 39°C;
(b) cooling the fermentation medium before fed batch conditions are attained to a temperature below about 25°C; and
(c) harvesting the host cells before fed-batch conditions are attained.

Examples of particular temperatures are those mentioned above. Thus cooling may be to a temperature in the range from about 10°C (or below) to about 25°C.

As mentioned above, yeast extract may be added. Such methods include those in which the above processes are used to cultivate a host capable of expressing ricin A, or an analogue thereof, in a growth medium and in which a supplement which includes yeast extract is added to the growth medium during the cultivation.

The yeast extract may be added by way of a single batch, a series of aliquots or a substantially continuous feed. The yeast extract will, in general, be added during the growth phase of the host. It is preferred, for example, that addition of the supplement which includes yeast extract is initiated at a predetermined time after the start of the cultivation. A particular example of this time is a point which is before fed-batch conditions are attained.

The rate of addition of the supplement which comprises yeast extract is preferably such that the growth medium does not become exhausted of yeast extract. Thus if the initial composition of the growth medium includes yeast extract then the addition of the supplement is preferable initiated before the yeast extract present in the initial composition has been consumed. Suitable rates of yeast extract feed include, for example, feed rates in the range of about 0.85 to 3.4 g.l-¹h-¹. A particular rate of feed is one in the range 1 to 2 g.l-¹h-¹, more particularly 1.7 gl-¹h-¹.

As mentioned above, the host cells may, in general, comprise bacterial cells, such as E.coli; and it is preferred for example, that the host cells are capable of expressing ricin A.

The fermentation is generally carried out at a temperature which favours growth of the host. A particularly suitable temperature which favour growth of bacterial cells, such as E. coli, is about 37°C. The temperature may be reduced during the cultivation (especially during the terminal portion of the cultivation) as mentioned above.

It has also been found that E.coli strain DS410 is surprisingly efficient at producing soluble ricin A. Also as mentioned above, it is generally preferred that the pH is adjusted and/or the temperature reduced.

After harvesting, the cells are processed to recover the ricin A. This processing will normally involve disrupting the cells, separating the crude ricin A polypeptide from other proteinaceous material by one or more extraction steps and further purifying the ricin A by gel filtration, high performance liquid chromatography or other conventional purification techniques.

The processes of the present invention have been found to be advantageous in the preparation of soluble polypeptides.

In general the processes of the present invention are advantageous in the generation and/or recovery of polypeptides in a soluble form. In particular it has been found that high yields of soluble polypeptide may be obtained by adjusting the pH of the growth medium during the process. It has also been unexpectedly found that if the growth medium is cooled during the terminal portion of the cultivation and the cells harvested during this terminal portion, then high yields of soluble polypeptide may be recovered. This cooling step has been found to be particularly advantageous since it leads to greater process latitude in that high yields of soluble polypeptide may be obtained even if there is a delay before harvesting. Fed-batch processes in which the pH is adjusted and an optional cooling step is effected have been found to be particularly advantageous.

In processes where the polypeptide is ricin A, the proceeses of the present invention have been found to be particularly advantageous.

The yields of soluble ricin A obtained have been found to be enhanced if yeast extract is added during the process. Processes in which the pH is also been adjusted, and especially fed-batch processes in which the pH is adjusted have been unexpectedly found to give high yields of soluble ricin A.

Also E.coli DS410 has been unexpectedly found to be advantageous in the preparation of soluble ricin A. It has also been found that the yield is enhanced if yeast extract is added during the process.

The invention will now be further described, by way of example only, with reference to the accompanying Examples and drawings in which:
- Figure 1: illustrates the construction of pICI 0020;
- Figure 2: illustrates the construction of pTB344;
- Figure 3: illustrates the construction of pICI 0042;
- Figure 4: illustrates the construction of pICI 1079;
- Figure 5: illustrates the construction of pICI 1187;
- Figure 6: illustrates a Coomassie blue-stained SDS gel of E.coli lysates in which track A is pICI 1102; B is pICI 0020, and C is molecular weight markers.
- Figure 7: illustrates a gel profile of pICI 1102 in which peak R represents ricin A:
- Figure 8: is a western blot of ricin A produced by pICI 1102 and in which track 1 is molecular weight markers; 2 and 3 are non-ricin producing clones; 4 is pICI 1102, and 5 is pICI 0020 (control plasmid-non ricin A sequence);
- Figure 9: is a partial sequence of pICI 1102;
- Figure 10: illustrates the construction of pICI 1102;
- Figure 11: describes a fragment used in the preparation of plasmids;
- Figure 12: is a plasmid map of pICI 0042;
- Figure 13: illustrates the seqeunce of a transcription terminator;
- Figure 14: is a plasmid map of pICI 1079;
- Figures 15, 16 and 17: illustrate the effect of fed batch fermentation on biomass, total ricin A accumulation and ricin A partitioning in the soluble fraction respectively for DS410;
- Figures 18, 19, 20 and 21: illustrate the effect of different pH control regimes on biomass concentration, total ricin A accumulation, percentage of soluble ricin A partitioning in the soluble fraction, and the yield of soluble ricin A respectively.
- Figure 22: illustrates the pH profiles employed; and
- Figures 23, 24 and 25: illustrate the effect of cooling.

### Growth Medium

### Composition of LCM50

| | Made up of distilled water g/l |
|---|---|
| KH₂PO₄ | 3.0 |
| Na₂HPO₄ | 6.0 |
| NaCl | 0.5 |
| Casein Hydrolysate (Oxoid L41) | 2.0 |
| (NH₄)₂SO₄ | 10.0 |
| Yeast Extract (Difco) | as specified |
| Glycerol | 35.0 |
| MgSO₄. 7H₂O | 0.5 |
| CaCl₂. 2H₂O | 0.03 |
| Thiamine | 0.008 |
| FeSO₄/Citric Acid | 0.04/0.02 |
| Trace element solution (TES) | (0.5ml l⁻¹) |
| Tetracycline | (10mg l⁻¹) |

The concentartion of yeast extract in the LCM50 growth medium is specified in each Example.

The trace element solution (TES) has the following composition:-

| | mg/10 ml deionised water |
|---|---|
| AlCl₃.6H₂O | 2.0 |
| CoCl₂.6H₂O | 0.8 |
| KCr(SO₄)₂.12H₂O | 0.2 |
| CuCl₂.2H₂O | 0.2 |
| H₃BO₃ | 0.1 |
| KI | 2.0 |
| MnSO₄.H₂O | 2.0 |
| NiSO₄.6H₂O | 0.09 |
| Na₂MoO₄.2H₂O | 0.4 |
| ZnSO₄.7H₂O | 0.4 |

### Strains

The E.coli strains referred to herein are freely available. For example, E.coli MM294, and W3110 are freely available. MM294 and W3110 may, for example be obtained from the E.coli Genetic Stock Centre, Yale University, USA. Δlac derivatives of W3110 are readily produced by those experienced in the art.

E.coli DS410 is well known (Dougan and Sherratt, Molecular and General Genetics, Vol 151, p151-160, 1977) and has the published genotype F⁻ara azi ton A lac Y min A min B rps L mal A xyl mtl thi. This strain is freely available to the public, and moreover was deposited by the Applicants on 7 June 1985, under the Budapest Treaty, with the National Collections Of Industrial & Marine Bacteria Ltd, Aberdeen, Scotland under deposition number 12100.

### EXAMPLE 1

E.coli strains MM294, DS410, and a Δlac derivative of W3110 termed W3110Δlac were transformed with pICI 1187.

The resultant strains MM294 (pICI 1187), DS410 (pICI 1187) and W3110Δlac (pICI 1187) were purified and maintained in glycerol stocks at -80°C. Aliquots of each culture were removed from stock and streaked onto agar plates of L-tetracycline to separate single colonies after overnight growth at 37°C.

Single colonies of DS410 (pICI 1187) W3110Δlac (pICI 1187) and MM294 (pICI 1187) were removed, separately resuspended in a 10 ml L-tetracycline broth and 100µl immediately inoculated into each of ten 250 ml Erlenmeyer flasks containing 75 ml of L-tetracycline broth. After growth for 16 h at 37°C on a reciprocating shaker the contents of the flasks in each set of ten were pooled and used to inoculate three separate fermenters containing LCM50 (10g/l yeast extract).

Fermentations were carried out at a temperature of 37°C and pH, controlled by automatic addition of 2M sulphuric acid and 6M sodium hydroxide solution, of pH6.7. The dissolved oxygen tension (dOT) set point was 50% air saturation and was initially controlled by automatic adjustment of the fermenter stirrer speed. Airflow to the fermenters was 20 L/min corresponding to 1 volume volume per minute (VVM) throughout.

Fermentations were performed for 14.25 hours and during that time samples were taken for measurement of optical density (OD₅₅₀), cell dry weight, accumulation and partitioning of ricin A within the cells. Ricin A accumulation was measured by scanning Coomassie blue stained SDS-PAGE gels of whole cell lysates of the sampled bacteria as is well known in the art. Partitioning of ricin A in the cytoplasmic (soluble) and inclusion body (insoluble) fractions of cells was determined by subjecting sampled bacteria to sonication lysis as is well known in the art.

Changing the E.coli host strain for plasmid pICI 1187 produced an unexpected change in the level of ricin A partitioned in the soluble cytoplasmic fraction. Table 1 illustrates the biomass yield, total ricin A accumulation and percentage of ricin A partioning in the soluble fraction at the end of the fermentation (14.25 hours) for strains DS410 (pICI 1187), W3110Δlac (pICI1187) and MM294(pICI 1187).

**Table 1**

| STRAIN | BIOMASS gl⁻¹ | TOTAL RICIN A ACCUMULATION % TMP * | % RICIN A PARTITIONING IN SOLUBLE FRACTION |
|---|---|---|---|
| DS410 (pICI 1187) | 9.3 | 5.9 | >80 |
| W3110Δlac (pICI 1187) | 14.4 | 7.5 | ca. 40 |
| MM294 (pICI 1187) | 10.4 | 7.6 | ca. 40 |

| | | | |
|---|---|---|---|
| * TMP means total microbial protein. | | | |

It is evident that by changing the E.coli host strain dramatic changes in ricin A partitioning can be produced.

### EXAMPLE 2

The fermentation process described in Example 1 was repeated with LCM50 growth medium (20g/l yeast extract) using strains DS410 (pICI 1187), W3110Δlac (pICI 1187) and MM294 (pICI 1187). A solution of yeast extract (333 gl⁻¹) was fed into the fermenters from 4.25h post fermenter inoculation at 0.85gl⁻¹h⁻¹. The yeast extract feed rate was increased to 1.7gl⁻¹h⁻¹ 5.75h post fermenter inoculation. When the carbon source in the fermentations became exhausted (leading to a rapid rise in dOT), a feed containing glycerol (714gl⁻¹) and ammonium sulphate (143gl⁻¹) was pumped into the fermenters at a rate which restricted bacterial oxygen uptake rate.

Except for the elevated batch yeast extract concentration and the supply of yeast extract solution during fermentation, the medium and fermentation conditions for the three fermenters were identical to the process described in Example 1.

The fermentation process described in this example resulted in an improvement in biomass of all 3 strains, an improvement in the amount of ricin A which was partitioned in the soluble fraction of strains W3110Δlac (pICI 1187) and MM294 (pICI 1187) and an improvement in the total yield of soluble ricin A of all strains compared to Example 1. Table 2 shows the final biomass concentrations, total ricin A accumulation, percentage of ricin A partitioning in the soluble fraction and calculated yields of soluble ricin A at the end of the fermentations (16.25 hours).

**Table 2**

| STRAIN | BIOMASS gl⁻¹ | TOTAL RICIN A ACCUMULATION (% TMP) | % RICIN A PARITIONING IN SOLUBLE FRACTION | YIELD SOLUBLE RICIN A mgl⁻¹* |
|---|---|---|---|---|
| DS410 (pICI1187) | 24.3 | 7.0 | 80 | ca 600(1) |
| W3110Δlac (pICI1187) | 23.1 | 8.4 | 60 | ca 600(1) |
| MM294 (pICI1187) | 19.3 | 9.7 | 50 | ca 400(1) |

| | | | | |
|---|---|---|---|---|
| * calculated yield mg soluble ricin A per litre fermentation broth. | | | | |
| (1) compared to calculated yields of soluble ricin A for the three strains in the fermentation processes described in Example 1 of ca 150-200mgl⁻¹. | | | | |

Similar results were obtained when the yeast extract concentration in LCM50 was 10 g/L and yeast extract was fed into the fermentor and/or when yeast extract was pumped into the fermenter at 0.85 gl⁻¹h⁻¹, 1.7 gl⁻¹h⁻¹ or 3.4 gl⁻¹h⁻¹ throughout.

Yeast extract feeding is thus able to increase the % of ricin A partitioning in the soluble fraction and the yield of soluble ricin per litre of fermentation broth.

### EXAMPLE 3

The fermentation process of Example 1 was repeated with E.coli strains MM294 (pICI 1187), W3110Δlac (pICI 1187) and DS410 (pICI 1187) using LCM 50 (10g/l yeast extract) supplemented with 100mgl⁻¹ of L-tryptophan. An L-tryptophan solution (10gl⁻¹) was pumped into the fermenters at 90mgl⁻¹h⁻¹ throughout the fermentations.

Table 3 compares the biomass concentration, total ricin A accumulation and percentage of ricin A partitioning in the soluble fraction at the end of the fermentation (14.25h) for the three strains. The supplement and feeding of L-tryptophan was carried out to influence the activity of the tryptophan promoter as is well known in the art. Compared with the fermentation process described in Example 1 and particularly Example 2 a detrimental effect was generally observed. Strain MM294 (pICI 1187) produces higher biomass levels but the percentage of ricin A partitioning in the soluble fraction is decreased. Strains DS410 (pICI 1187)) and W3110Δlac (pICI 1187) show no change in ricin A partitioning but the level of biomass produced is decreased. The results achieved with yeast extract described in Example 2 are thus most suprising since the added yeast extract contains large amounts of tryptophan.

**Table 3**

| STRAIN | BIOMASS gl⁻¹ | TOTAL RICIN A ACCUMULATION (% TMP) | % TOTAL RICIN A PARTITIONING IN SOLUBLE FRACTION |
|---|---|---|---|
| DS410 (pICI1187) | 7.6 | 6.8 | >80 |
| W3110Δlac (pICI1187) | 11.0 | 7.2 | ca. 40 |
| MM294 (pICI1187) | 20.8 | 7.2 | ca. 20 |

### EXAMPLE 4

The fermentation process of Example 2 was repeated with E.coli strain DS410 (pICI 1187) in LCM50 (20 g/l yeast extract).

The fermentation was carried out at a temperature of 37°C and pH, controlled by automatic addition of 2M sulphuric acid and 6M sodium hydroxide solution, of pH 6.7. The dissolved oxygen tension (dOT) set point was 50% air saturation and was initially controlled by automatic adjustment of the fermenter stirrer speed. Air flow to the fermenter was initially 20L/min corresponding to 1 volume volume per minute (VVM) and was increased manually to 45 L/min when the fermenter stirrer speed reached its maximum. The fermentation was performed for 34 hours and during that time samples were taken for measurement of optical density (OD₅₅₀), cell dry weight, accumulation and partitioning of ricin A within the bacterial cells. Ricin A accumulation was measured by scanning Coomassie blue stained SDS-PAGE gels of whole cell lysates of the sampled bacteria as is well known in the art. Partitioning of ricin A in the cytoplasmic (soluble) and inclusion body (insoluble) fractions of cells was determined by subjecting sampled bacteria to sonication lysis as is well known in the art. A solution of yeast extract (333 gl⁻¹) was pumped into the fermenter 4.5 hours post inoculation at 1.7 g 1⁻¹ h⁻¹.

Between 12-13 hours post inoculation the supply of carbon-source in the fermentation became exhausted leading to a rapid rise in dOT from 50% air saturation. From this point, a feed containing glycerol (714 gl⁻¹) and ammonium sulphate (143 gl⁻¹) was pumped into the fermenter at a rate which restricted the bacterial oxygen uptake rate (OUR) to approximately 80% of the fermenter's maximum oxygen transfer rate (OTR) whilst returning and then maintaining the dOT at 50% air saturation.

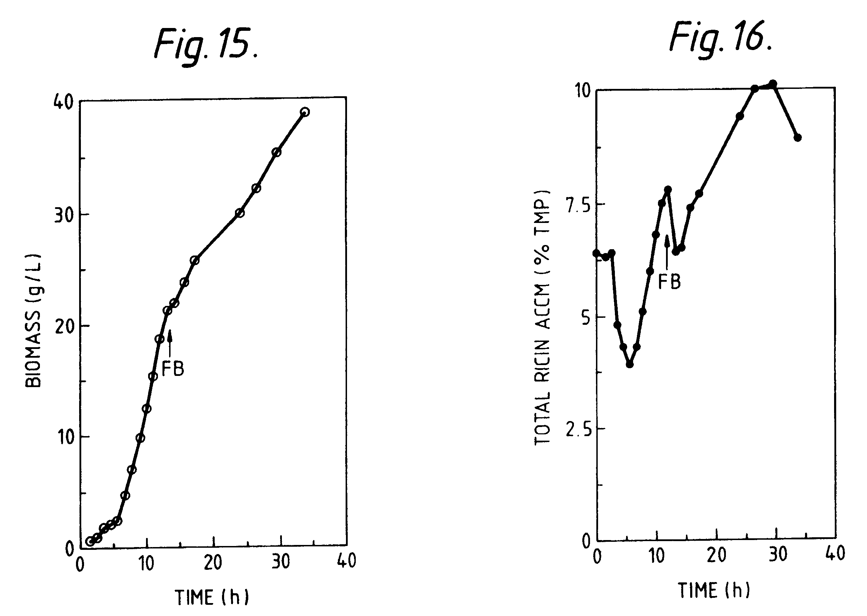

The influence of fed-batch fermentation on biomass production, total ricin A accumulation and ricin partitioning in the soluble fraction is illustrated in Figures 15, 16 and 17 respectively. The point at which fed-batch fermentation commences is indicated on each figure (FB). The effect of fed-batch fermentation on ricin portioning in the soluble fraction is clear.

### EXAMPLE 5

A single vial of DS410 (pICI 1187) was removed from stock and 100µl of the culture removed and immediately inoculated into each of three 2 litre Erlenmeyer flasks containing 600ml of L-tetracycline broth. After growth for 16h at 37°C on a reciprocating shaker the contents of the flasks were used to inoculate three fermenters containing LCM50 growth medium (20g/l yeast extract).

The fermentations were caried out at a temperature of 37°C and pH, controlled by automatic addition of 2M sulphuric acid and 6M sodium hydroxide solution, of:
(A) pH 6.7 throughout the fermentation
(B) pH 6.7 up to 10h post inoculation, thereafter controlled at pH6.0
(C) pH 6.7 up to 10h post inoculation, thereafter controlled at pH7.6.
The dissolved oxygen tension (dOT) set point was 50% air saturation and was initially controlled by automatic adjustment of the fermenter stirrer speed. Air flow to the fermenters was initially 20L/min corresponding to 1 volume volume per minute (VVM) and was increased manually to 45L/min when the fermenter stirrer speed reached its maximum (1000rpm). The air flow to the fermenters was decreased manually back to 20L/min when, towards the later stages of the fermentations, the stirrer speed had automatically decreased to approximately 500rpm.

The fermentations were performed for 23h and during that time samples were taken for measurement of optical density (OD₅₅₀), cell dry weight, total ricin A accumulation and ricin partitioning in the soluble fraction. Ricin A accumulation was measured by scanning Coomassie blue stained SDS-PAGE gels of whole cell lysates of the sampled bacteria as is well known in the art. Partitioning of ricin A in the cytoplasmic (soluble) and inclusion body (insoluble) fractions of cells was determined by subjecting sampled bacteria to sonication lysis as is well known in the art.

A solution of yeast extract (333 gl⁻¹) was fed into the fermenters from 4.5h post inoculation at 1.7gl⁻¹h⁻¹.

When the carbon source in the fermentations became exhausted (leading to a rapid rise in dOT from 50% air saturation) a feed containing glycerol (714 gl⁻¹) and ammonium sulphate (143 gl⁻¹) was pumped into the fermenters at a rate sufficient to meet the maximum carbon demand of the bacteria. The feed rate of the limiting carbon source and ammonium sulphate was then left unchanged during the rest of the fermentation.

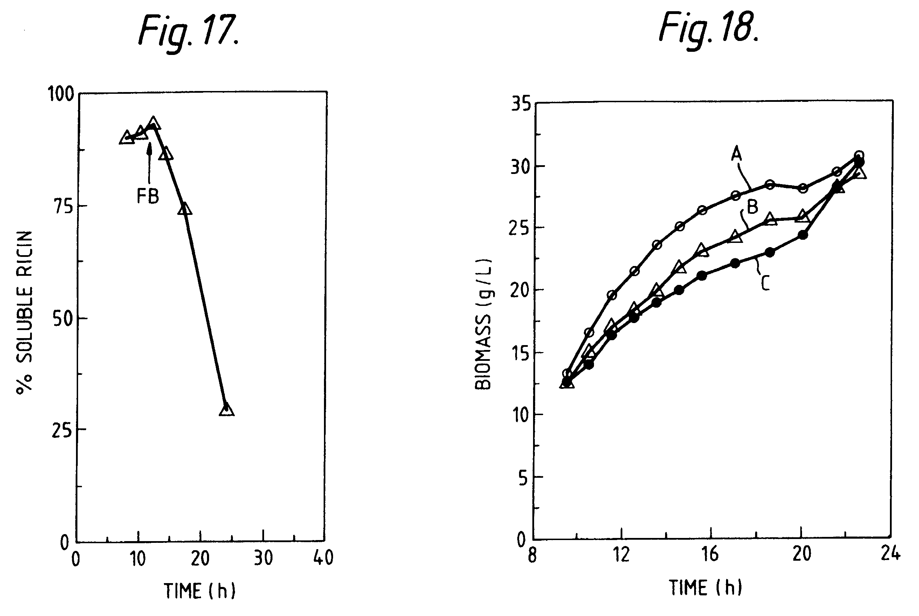

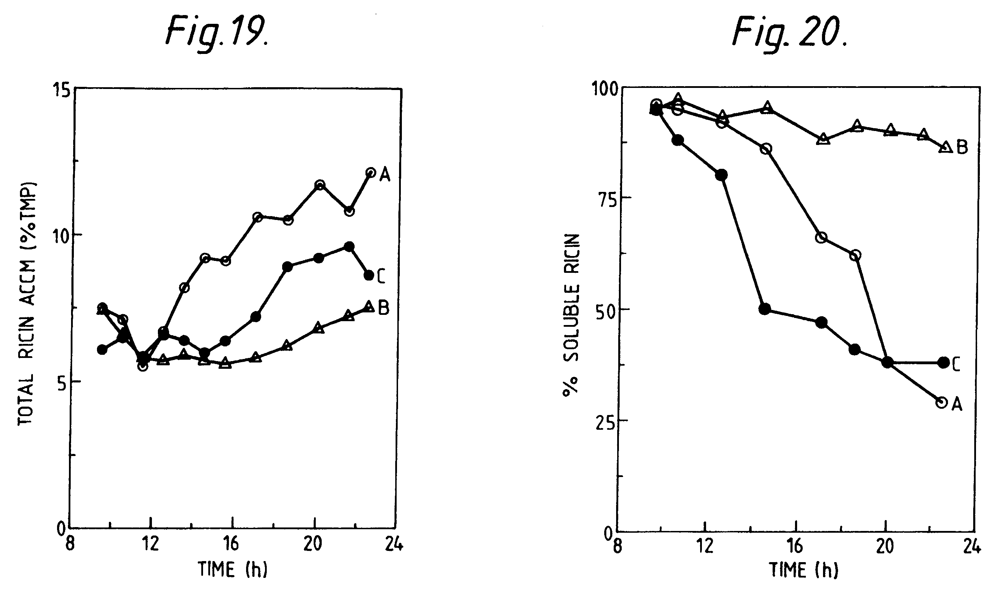

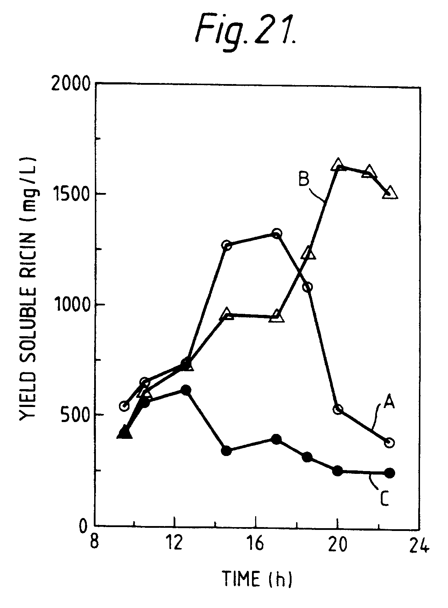

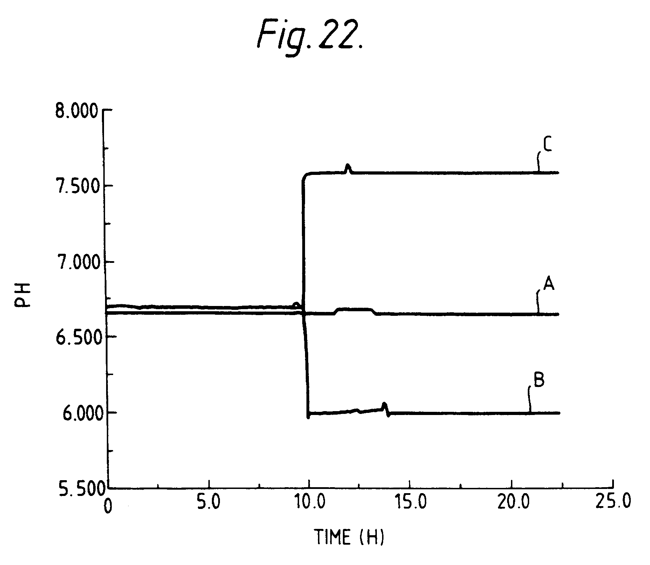

The effect of the three different pH control regimes (A, B and C) is shown in Figures 18, 19, 20 and 21 which show the biomass concentration, total ricin A accumulation, percentage of ricin A partitioning in the soluble fraction and calculated yield of soluble ricin A respectively. Figure 22 shows the pH of the growth medium during the fermentations. The advantageous effect on yield of soluble ricin of altering the pH for pH6.7 to pH6.0 is clearly illustrated.

### EXAMPLE 6

The fermentation process described in Example 4 was repeated, but 11.5 hours post fermenter inoculation the fermentation temperature was decreased gradually.

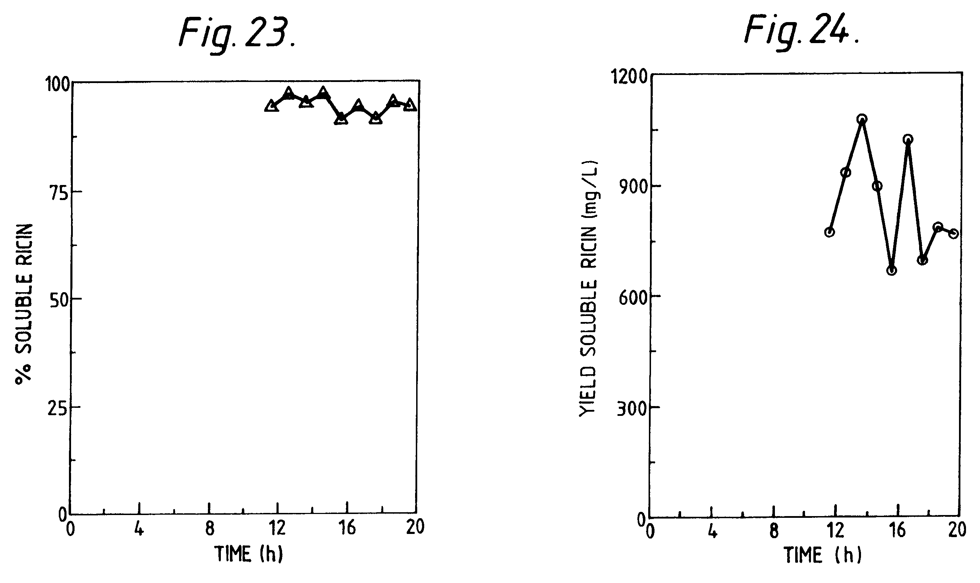

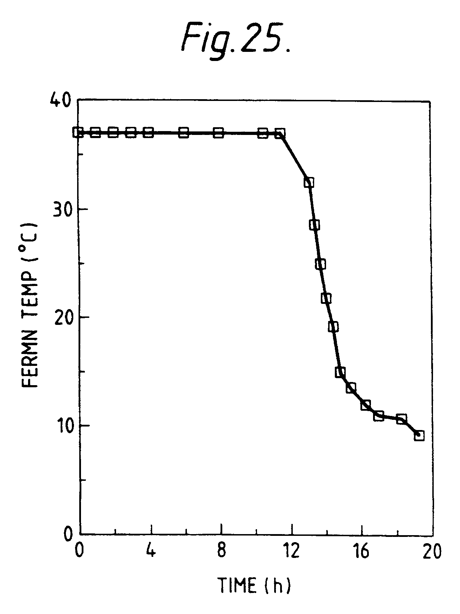

An unexpected maintenance of ricin solubility was observed. Figures 23, 24 and 25 illustrate the results obtained. Figure 25 shows the fermentation temperature profile during the process.

### PREPARATION OF PLASMIDS

The following illustrates the preparation of plasmid pICI 1187 used above. Several intermediate stages in the derivation of the vector used to prepare recombinant ricin A are described.

### EXPERIMENTAL PROCEDURES

### 1. Synthetic oligonucleotides

Synthetic oligonucleotides were used to introduce specific DNA sequence alterations of the ricin gene. All oligonucleotides subsequently described were prepared on an Applied Biosystems 380A DNA synthesiser from 5′-dimethoxytrityl base-protected nucleoside-2-cyanoethyl-N,N-diisopropylphosphoramidites and protected nucleosides linked to controlled-pore glass supports on a 0.2 micro mol scale, according to protocols supplied by Applied Biosystems Inc.

Each oligonucleotide, after cleavage from the solid support and removal of all protecting groups, was dissolved in water (1ml) and a measurement of absorbance at 260nm used to determine concentration.

### 2. Enzymes

A variety of restriction endonucleases and DNA modifying enzymes were used in the manipulations described below. These were purchased from one of a number of suppliers (Amersham International, Bethesda Research Laboratories, Boehringer Mannheim or New England Biolabs) and used in accordance with the manufacturers instructions with respect to reaction conditions.

### 3. Geneclean (TM)

The kit contains 1) 6M sodium iodide 2) a concentrated solution of sodium chloride, Tris and EDTA for making a sodium chloride/water ethanol/water wash; 3) Glassmilk (TM)- a 1.5 ml vial containing 1.25 ml of a suspension of silica matrix in water.

This is a technique for DNA purification based on the method of Vogelstein and Gillespie published in Proceedings of the National Academy of Sciences USA (1979) Vol 76, p 615.

Alternatively any of the methods described in "Molecular Cloning - a laboratory manual" Second Edition, Sambrook, Fritsch and Maniatis (Cold Spring Harbor Laboratory, 1989) can be used.

### 4. Sequenase (TM)

Chemically modified T7 DNA polymerase.

Based on the procedure of Tabor and Richardson published in "Proceedings of the National Academy of Sciences USA (1987) vol 84 pp 4767-4771.

### 5. Construction of the pICI exprerssion vectors

### 5.a) pICI 0020

Plasmid vector pICI 0020 is a pAT153 based plasmid in which the 651 bp EcoRI-AccI region is replaced by a 167 bp EcoRI - ClaI fragment consisting of:-
(1) a synthetic E.coli trp promoter and trp leader ribosome binding site
(2) a translation initiation codon
(3) a multiple restriction enzyme recognition sequence derived from M13mp18, containing sites for KpnI, BamHI, XbaI, SalI, PstI, SphI and HindIII
(4) a synthetic transcription termination sequence

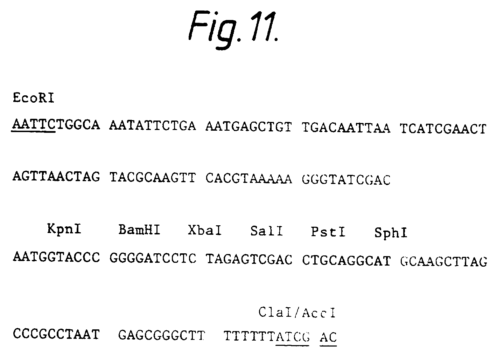

The DNA sequence of this region is shown in Figure 11.

The construction of a plasmid vector containing a synthetic trp promoter sequence is published (Windass et al Nuc.Acids Res. 10 p6639-6657, 1982). A promoter fragment was isolated from such a vector after digestion with the enzymes EcoRI and HpaI and purification of the appropriate band from an agarose gel by electro-elution (in "Molecular Cloning - A Laboratory Manual", Maniatis, Fritsch and Sambrook, published by CSH laboratory, second edition 1989 and hereinafter referred to as "Maniatis").

A pair of complementary synthetic oligonucleotides were prepared which would ligate to the HpaI end of the promoter fragment providing the natural trp leader ribosome binding site, a translation initiation codon and a 3′ KpnI cloning site. These oligonuleotides were mixed in equimolar concentrations and allowed to anneal by heating to 100°C followed by slowly cooling to room temperature.

The promoter fragment and annealed oligonucleotides were then ligated and the appropriate band isolated from a polyacrylamide gel by electroelution. This fragment was then ligated with an M13mp18 vector derivative containing the trp attenuator sequence (generated from synthetic oligonucleotides) cloned into the HindIII site and introducing an additional ClaI restriction site 3′ to the attenuator. The ligated DNA was transfected into E.coli strain JM109 (Yanisch-Perron et al Gene, 33, p103, 1985) made competent by the CaCl₂ method (Maniatis, chapter 1p82). After plating out and incubation of the plates, plaques were screened by the method of Benton and Davies (Maniatis, chapter 4p41) using a ³²P labelled probe generated by nick translation of the EcoRI-HpaI promoter fragment isolated previously. Single stranded DNA was prepared from positively hybridising plaques by a standard method (Maniatis, chapter 4p29) and sequenced using the M13 universal primer and the Sanger dideoxy chain termination method as provided in kit form by a number of suppliers eg. Sequenase (United States Bioscience).

RF DNA was prepared from one isolate in which the promoter/ribosome binding site/attenuator sequence had been confirmed. This DNA was digested with EcoRI and ClaI and the appropriate fragment isolated from a polyacrylamide gel as above. Plasmid pAT153 was digested with the enzymes EcoRI and AccI and ligated with the isolated promoter fragment. Ligated DNA was used to transform competent E.coli HB101 (Bethesda Research Laboratories) and ampicillin resistant colonies selected.

Plasmid DNA from several clones was prepared and DNA sequence derived from the region between the EcoRI and ClaI sites. One clone confirmed as containing the correct promoter/attenuator region was named pICI 0020.

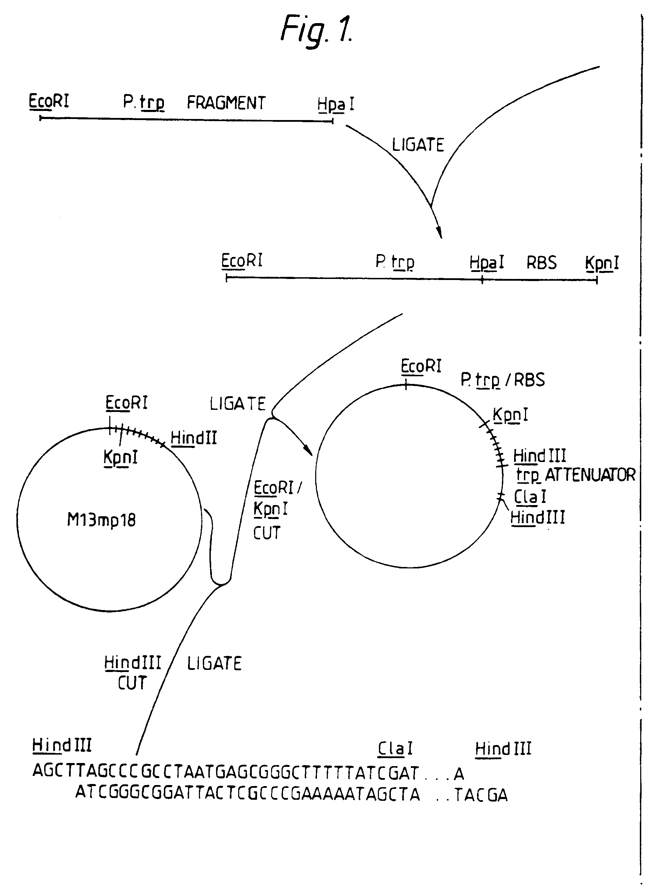

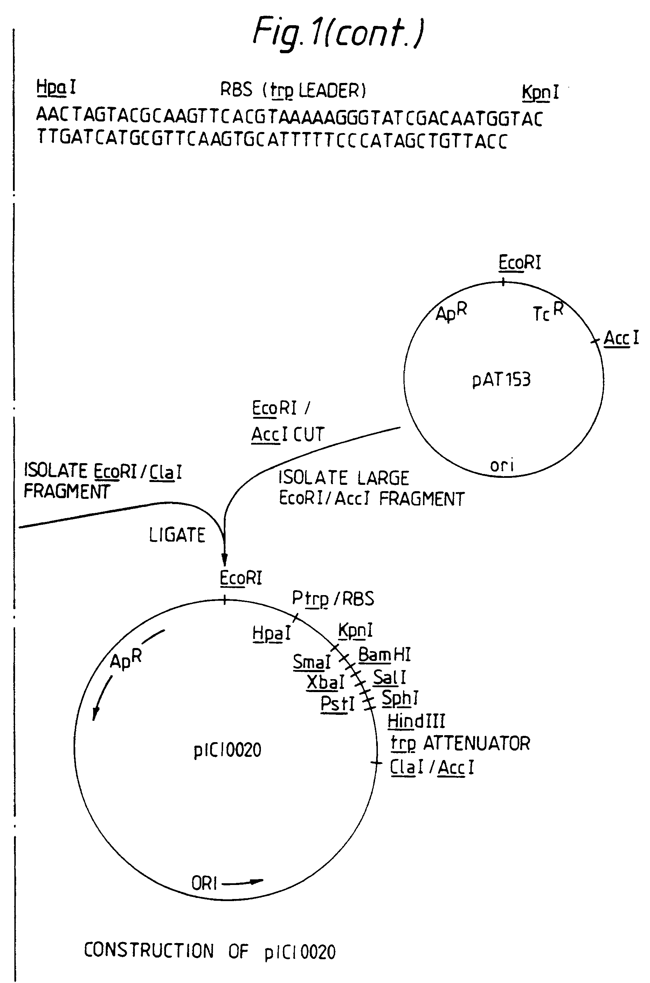

This construction is outlined in fig.1.

### 3.b) pICI 0042

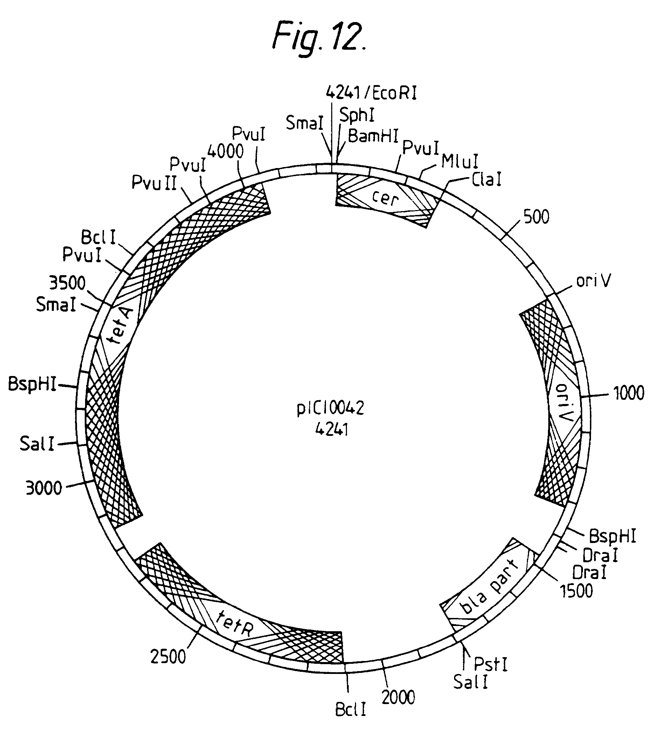

pICI 0042 (Figure 12) is a plasmid in which the antibiotic resistance markers of pAT153 have been replaced by a single, inducible tetracycline resistance gene from the plasmid RP4 (encoded by the gene tetA and regulated by the product of the tetR gene). These genes have been characterised by Klock et al (J.Bacteriol. 161 p326-332, 1985). A plasmid stability function (cer) has also been incorporated. This obviates the requirement for β-lactam antibiotics in any part of the production process and will also allow assays for these in the final product. Because the new resistance marker is only expressed in the presence of antibiotic, the tetA gene product will not be a potential contaminant of recombinant ricin A in cultures where the plasmid is stabily maintained in the absence of tetracycline.

The initial stage in the generation of this vector was to produce a derivative of pAT153 from which the gene encoding tetracycline resistance had been completely removed. A complementary pair of synthetic oligonucleotides were designed to replace the EcoRI-AvaI fragment from pAT153 with a short sequence containing several unique restriction endonuclease sites for subsequent cloning.

pAT153 plasmid DNA was digested with the enzymes EcoRI and AvaI and the 2.175Kbp plasmid DNA fragment isolated from a 0.7% agarose gel using Geneclean (Bio 101, California) in accordance with the manufacturers instructions. The 1.425Kbp fragment containing the tetracycline resistance gene is thus removed.

The oligonucleotides (exp79 and 80) were phosphorylated using T4 polynucleotide kinase and equimolar amounts annealed together. A sample of the annealed oligonucleotides was then ligated with the plasmid fragment from pAT153. Ligated DNA was transformed into E.coli HB101 (BRL) and ampicillin resistant colonies selected.

Several colonies were picked for small-scale plasmid DNA preparation (method of Birnboim and Doly as specified in Maniatis, chapter 1p25) and the desired construction identified by restriction analysis with suitable enzymes eg. EcoRI AvaI and BamHI. The structure of 3 isolates identified as having the correct restriction pattern was confirmed by DNA sequence analysis using a pBR322 EcoRI site clockwise primer (New England Biolabs). One isolate was named pICI 0019.

RP4 plasmid DNA was isolated from extant stocks by the method of Holmes and Quigley (Maniatis, chapter 1p29). This DNA was cut to completion with BglII and then partially with XmaI (at 25°C for up to 35min) taking samples at various timepoints until a 2.45 Kbp fragment containing the tetR and tetA was clearly identifiable. A sample of pUC8 DNA (Amersham International) was digested to completion with BamHI and XmaI. Ligations were performed to insert the tetracycline resistance genes into the pUC8. Ligated DNA was used to transform E.coli C600 (Appleyard, R.K. Genetics 39 p440, 1954) made competent by the CaCl₂ method (Maniatis, chapter 1p82) and tetracycline resistant colonies selected. Plasmid DNA was prepared from 8 clones (Holmes and Quigley) and the presence of the RP4 tetR and A genes confirmed by restriction analysis. One of these isolates was named pTB344.

The tetracycline resistance genes were then inserted into pICI 0019 (described above) by replacement of an EcoRI/PstI fragment from pICI 0019 with the corresponding fragment from pTB344. This results in replacement of the majority of the ampicillin resistance gene in pICI 0019 with the tetracycline resistance genes. After digestion and ligation of the plasmid DNAs, followed by transformation of E.coli C600, colonies were selected on the basis of phenotype ie. Tc^{R} and Ap^{S}. Plasmid DNA was prepared from 4 such clones and digested with a combination of enzymes eg. BamHI/PstI/SstI, EcoRI/SalI SmaI, StyI/SalI and AvaI/PstI. All 4 clones produced restriction patterns consistent with the desired construct. One of these was designated pTB351.

Summers and Sherratt (Cell 36 p1097-1103, 1984) have shown that the instability of plasmids derived from ColEI (eg. pAT153) is due to the loss of a 283bp sequence, cer, present in the parent plasmid. This sequence helps prevent the formation of plasmid oligomers, the latter appearing to disrupt plasmid partitioning in some as yet undefined way. The cer sequence (Summers, D. et al MGG 201, p334-338, 1985) was kindly provided by Prof.D.Sherratt in the form of a fragment cloned into pUC18 (pKS492). pKS492 plasmid DNA was digested with BamHI and TaqI to release a 289bp cer-containing fragment. Plasmid pTB351 DNA (isolated from the dam⁻ host E.coli GM48 - Arraj, J.A. and Marinus, M.G. J.Bact. 153 p562-565, 1983) was digested to completion with BamHI and ClaI and ligated with the digested pKS492 DNA. After transformation of competent E.coli C600 with ligated DNA, tetracycline resistant colonies were selected. Restriction analysis of plasmid DNA from putative clones with the enzymes AvaI, MluI and PvuI was used to confirm the presence of cer. One isolate with the correct structure was named pICI 0042.

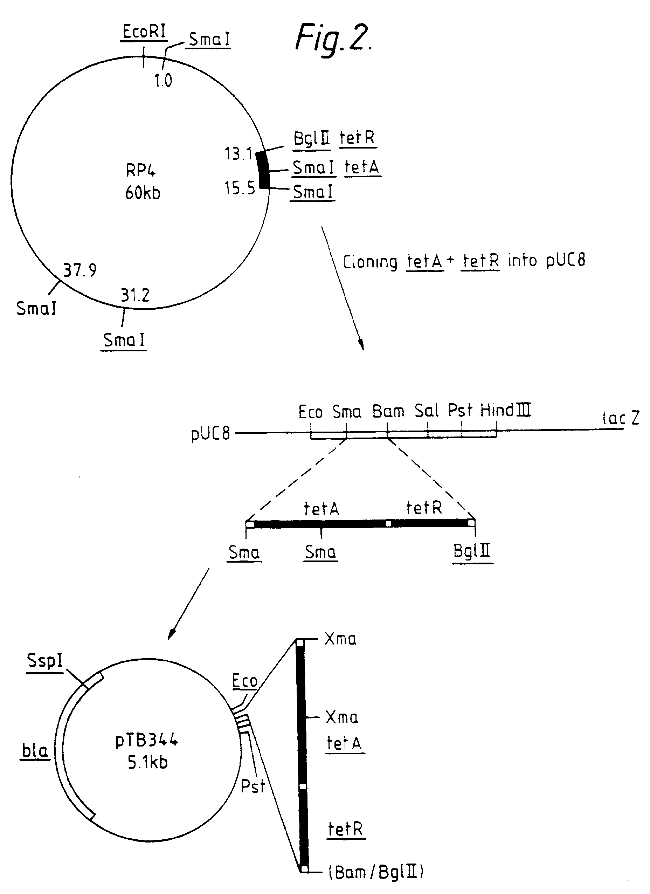

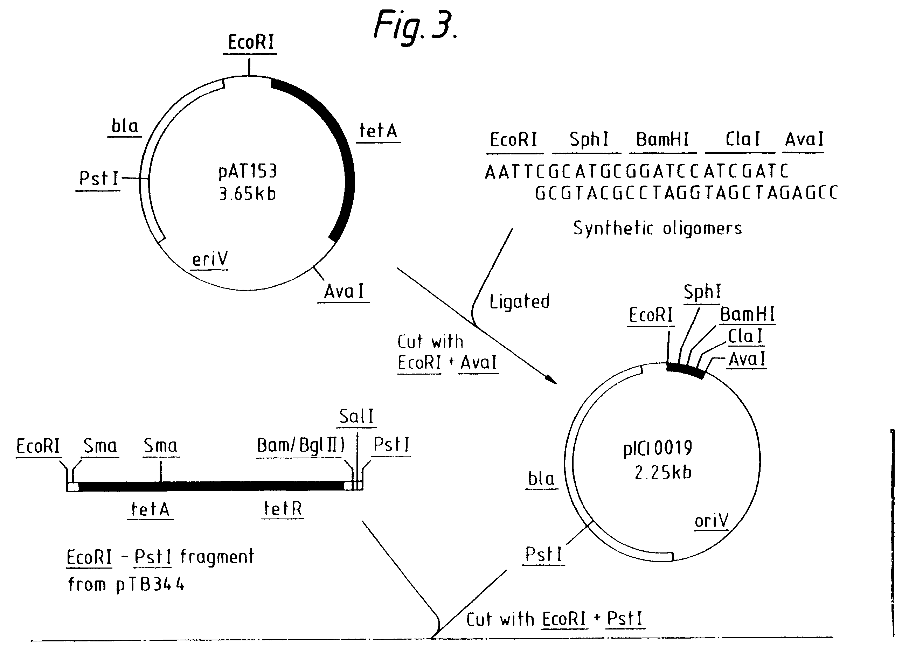

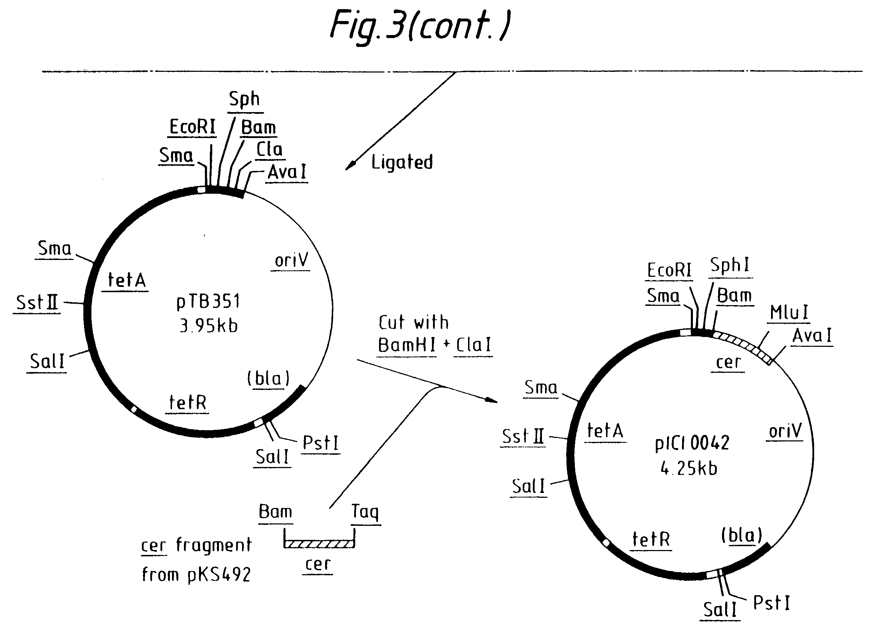

The construction of these plasmids is outlined in figs.2 and 3.

### 5.c) pICI 1079

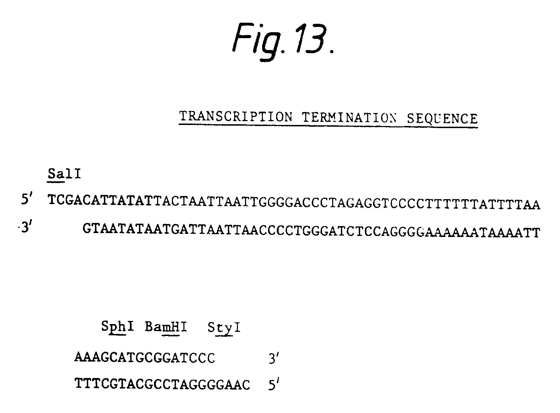

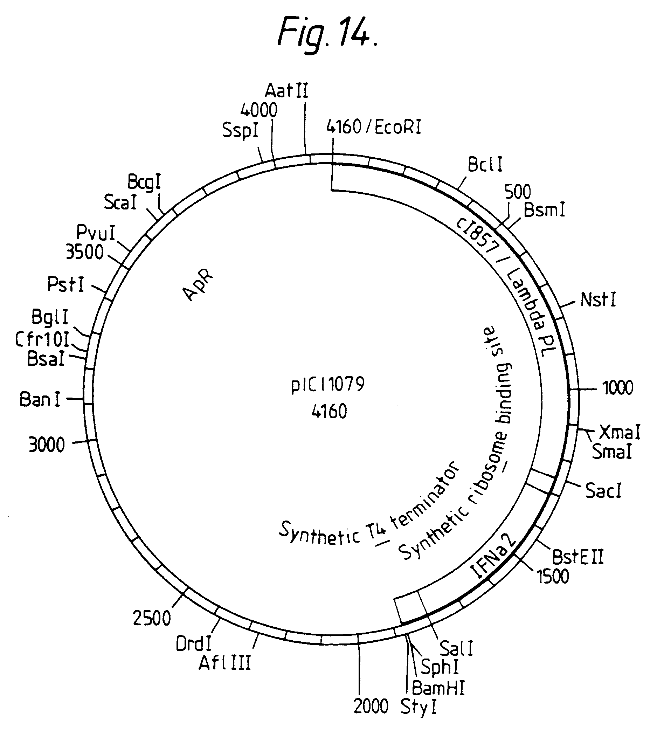

Plasmid vector pICI 1079 is an ampicillin resistant, pAT153-derived plasmid containing the following elements between the EcoRI and StyI restriction sites:-
(i) a CI857 gene from phage λ;
(ii) a λP_{L} promoter;
(iii) a synthetic ribosome binding site;
(iv) a synthetic interferon α2 gene sequence;
(v) a synthetic transcription terminator sequence, derived from phage T4, between the SalI and StyI restriction sites. The DNA sequence of this transcription terminator is shown in Figure 13.
   pICI 1079 is illustrated in Figure 14.
   pICI 1079 has been deposited under the Budapest Treaty. The deposit has been made at the NCIMB, 23 St Machaer Drive, Aberdeen, Scotland.

This plasmid was used to provide a source of the T4 transcription terminator for the generation of the ricin A expressing clone pICI 1185 (see 7.d below). The starting point for the generation of this plasmid was pICI 1043. pICI 1043 is a plasmid based on pICI 0020 (see 3.a above) in which an expression cassette containing a λP_{L} promoter and interferon α2 gene (Edge et al Nuc.Acids Res. 11 p6419-6435, 1983) is present between the EcoRI and SalI sites.

A complementary pair of oligonucleotides was synthesised to generate the transcription terminator from gene 32 of bacteriophage T4 with 5′ SalI and 3′SphI cohesive ends. This fragment was ligated with a plasmid fragment isolated from pICI 1043 which had been digested to completion with SalI and SphI. The intermediate plasmid thus produced (pICI 1078) contained both the T4 terminator and trp attenuator sequences in tandem.

A second pair of complemetary oligonucleotides was then used to replace the trp attenuator sequence (and remaining part of the tetracycline resistance gene) by insertion between the SphI and StyI sites of pICI 1078. A unique BamHI site was introduced within this synthetic fragment.

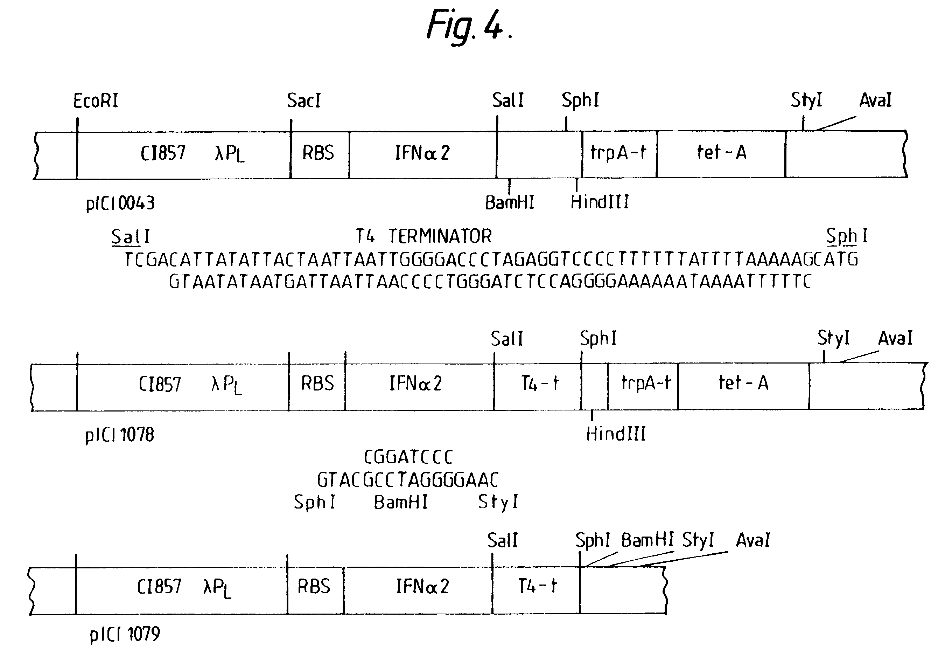

These manipulations are outlined in fig.4.

### 6. Generation of a ricin A expressing clone

### 6.a) Preparation of pUC8RA plasmid DNA

A clone (pUC8RA) was generated which contains the cDNA for ricin A. This clone contains A-chain cDNA from base number -74 in the leader sequence through to the BamHI site within the B-chain (base number 857) according to the published cDNA sequence (Lamb,I.F., Roberts,L.M., Lord,J.M. Eur.J.Biochem , 1985, 148, p265-270) in plasmid pUC8 (Vieira,J and Messing,J. Gene, 19, p259, 1982). In addition, site-directed mutagenesis has been used to generate a translation termination codon immediately 3′ to the final codon of mature ricin A (as reported in O'Hare, M et al FEBS Letts, 1987, 216, p73-78). The entire A-chain coding region is included in a BamHI fragment from this clone.

A small quantity of pUC8RA plasmid DNA was obtained from the originators. For future stocks, a dilution of this DNA was used to transform E.coli DH5α competent cells (Bethesda Research Laboratories) and an ampicillin resistant transformant selected. Plasmid DNA from this clone was prepared by a modified Birnboim-Doly procedure (Maniatis, chapter 1p25). Samples of this DNA were digested with BamHI and BanI separately and compared to corresponding digests of the original sample of DNA after electrophoresis on an agarose gel. No differences in restriction pattern were observed and, on this basis, the two DNA samples were assumed to be identical.

### 6.b) Sub-cloning into M13

BamHI digests of pUC8RA plasmid DNA and RF (replicative form) DNA from the phage M13 strain K19 (Anglian Biotechnology) were "shotgun" ligated using standard conditions (Maniatis, chapter 1p68). Control ligations were also performed. The ligated DNAs were used to transform E.coli strain TG1 (Gibson, 1984/Anglian) made competent by the CaC1₂ method (Maniatis, chapter 1p82).

The transformation frequences indicated efficient ligation and recombinant phage were expected in the progeny. Recombinant phage were predicted to produce clear plaques on IPTG + X-gal (BRL) containing plates due to disruption of the lacZ (β-galactosidase) gene. Wild type phage produce blue plaques due to conversion of the X-gal by β-galactosidase.

Several clear plaques were picked for single strand DNA preparation. Direct gel electrophoresis of lysed phage suspensions indicated that one phage clone contained a sizeable insert which was confirmed by sequencing to be the ricin A-chain coding sequence. Only 182 bases of the mature ricin A coding sequence were confirmed but this was taken as sufficient evidence for the presence of the entire ricin A gene. This clone was named M13K19RA.

### 6.c) Mutagenesis of M13K19RA

To generate a KpnI site, compatible with pICI expression vectors, at the start of mature ricin A, the following changes (underlined) are necessary:- 
5′....GATAACAACATATTCCCCAAA...... 3′    SEQ.ID NO 1
.....Ricin leader sequence ....|---Mature ricin A-->
 Changed to: 
5′....GATAACAACATGGTACCCAAA.......3′    SEQ.ID NO 2
KpnI
Translation initiation
 and result in an ATG codon overlapping a KpnI site. A KpnI fragment containing ricin A can be excised from the mutant and inserted into the ICI expression vector series . Two N-terminal amino acid modifications are made (ile-phe to met-val).

The single stranded DNA prepared from M13K19RA was the template for the mutagenesis step for each mutation strategy. A single oligonucleotide (DTR16) introducing all the mutational changes for this strategy was synthesised.

DTR16 5′ AACAACATGGTACCCAAACAA 3′ SEQ.ID NO 3

Several protocols exist for the introduction of specific DNA sequence changes by site directed mutagenesis. The procedures outlined below were achieved using the method of Eckstein et al (Nuc. Acid Res., 1985, 13 p8749-8764 and 1986, 14, p9679-9698) as provided in kit form (Amersham International) and used in accordance with the manufacturers instructions.

The principle of this method is to prime the single-stranded DNA template with the mutagenic oligonucleotide and synthesise the complementary strand incorporating dATPαS in place of dATP. Using this nucleotide results in the formation of phosphorothioate bonds which are not cleaved by certain restriction enzymes (eg. NciI). After synthesis of the second strand, NciI is used to nick the parent strand and exonuclease III added to digest back past the mutation point. DNA polymerase I then allows resynthesis of the parent strand. Consequently, the mutagenic oligonucleotide acts as a template for resynthesis and the mutation is introduced into both strands prior to transformation. Mutation frequencies up to 96% of the total progeny are claimed and screening is performed simply by picking plaques at random for DNA sequence analysis.

In our experiments 4 out of 4 plaques picked were correctly mutated.

Having chosen one mutant (MRA16), RF DNA was prepared and checked for the presence of the newly generated restriction fragment ie KpnI.

### 6.d) Cloning, Expression and Initial Characterisation

The pICI series of expression vectors (see section 5) can accept DNA fragments cloned into a unique KpnI restriction site adjacent to the Trp promoter. The KpnI site overlaps the translation initiation codon (ATG) which is situated 8bp downstream from the Shine-Dalgarno site (AGGA) of the promoter.

Having verified the sequence of MRA16, a large scale (∼5µg RF DNA) KpnI digest was performed and the relevant ricin A coding DNA fragment isolated from an agarose gel (Nu-Sieve GTG agarose, FMC Bio-products) by phenol extraction of an excised gel slice according to the manufacturer's protocol.

pICI 0020 (see 5a) was digested with KpnI and then dephosphorylated using calf intestinal alkaline phosphatase (CIP - Boehringer Mannheim). The latter treatment prevents recircularisation of the vector upon ligation which would lead to a high proportion of parentals in the transformation progeny.

Ligations were set up with ratios of plasmid vector to isolated fragment from 8:1 (w/w) to 1:3 for the various strategies. Control ligations to test the effectiveness of phosphatase treatment, ligase activity etc., were included. The ligation conditions were appropriate for the source of T4 DNA ligase used (New England Biolabs or Amersham). Reactions were generally incubated at 15°C overnight.

Fifty percent of each ligation (5µl) reaction was diluted to 100µl with 1 x TNE (50mM Tris, 50mM NaC1, 1mM EDTA) and 200µl of competent E.coli DS410 added. After a standard transformation protocol (Maniatis, chapter 1p74), the cells were plated onto L agar plus streptomycin (25µg/ml) and ampicillin (100µg/ml) and incubated at 37°C overnight. E.coli DS410 has a chromosomal streptomycin resistance gene.

The transformation plates were examined after incubation. In general, 5 to 10 times more colonies were seen in ligations compared to controls without ligase. In some cases, little difference in the number of colonies produced in the presence or absence of ligase occurred indicating incomplete digestion of the vector or poor ligase activity.

Transformants, plus the relevant controls were picked onto nitrocellulose filters placed on L agar plates for hybridisation screening (based on the method of Grunstein and Hogness as described in Maniatis, chapter 1p98). After incubation, the colonies were lysed in situ using 10% SDS and 1M NaOH, neutralised using 1M Tris (pH 7.5) and dried under vacuum at 80°C for 2 hours.

Hybridisation probes were generated by ³²p labelling of the mutational oligonucleotides using T4 polynucleotide kinase. The filters were probed at room temperature and then washed in stages up to 55-65°C to remove non-specifically bound counts before autoradiography. Specific hybridisation indicated putative clones containing ricin A DNA.

Small scale DNA preparations (by the methods of Holmes and Quigley or Birnboim-Doly as specified in Maniatis, chapter 1p25) were made from positively hybridising clones. The DNAs were digested with the relevant restriction enzymes eg. KpnI and EcoRI/BglII, and analysed by electrophoresis on agarose gels. Vector DNAs and mutated RF DNAs were cut with the same enzymes to demonstrate the fragment sizes expected for the correct clones.

Larger scale plasmid DNA preparations (Birnboim-Doly) of each clone were used for more detailed restriction analysis, eg. ClaI, HindIII, BamHI EcoRI/BglII KpnI, and ScaI. On agarose gels, these digests showed the size of fragment inserted, an indication of its orientation and the gain of some unique ricin A-chain enzyme sites.

### 6.e) Expression studies

The clones positively identified by hybridisation and restriction screening were tested for expression of ricin A by SDS-PAGE analysis of total cell lysates. The standard conditions for expression studies were:-
1) Inoculate 10ml of L-broth + antibiotic(s) with a single colony and grow at 37°C overnight with gentle shaking.
2) Take 750µ1 of the L-broth overnight and pellet the cells in a microfuge (1 min at 6500 rpm).
3) Resuspend pellet in 300µ1 M9 medium (Maniatis, appendix A.3) + 0.02% casein hydrolysate + 0.2% glucose + 50µg/ml thiamine and inoculate into 10ml of same.
4) Incubate for 7 hours or overnight at 37°C with gentle shaking.
5) After incubation, measure OD₅₄₀, pellet the cells and resuspend to OD₅₄₀ = 10 per ml in Laemmli sample buffer (Maniatis, chapter 18p53). Boil for 15 minutes.
6) Load 20µl of total cell lysate on an SDS polyacrylamide gel, electrophorese, stain with Coomassie blue, destain and visualise.

Of the clones studied by SDS-PAGE, only 1 showed an additional band with equivalent molecular weight of ∼29KD (equivalent to that estimated for unglycosylated, mature ricin A). Gel scans indicated the expression level to be in the range of 5-10% of total cell protein. This clone was named pICI 1102.

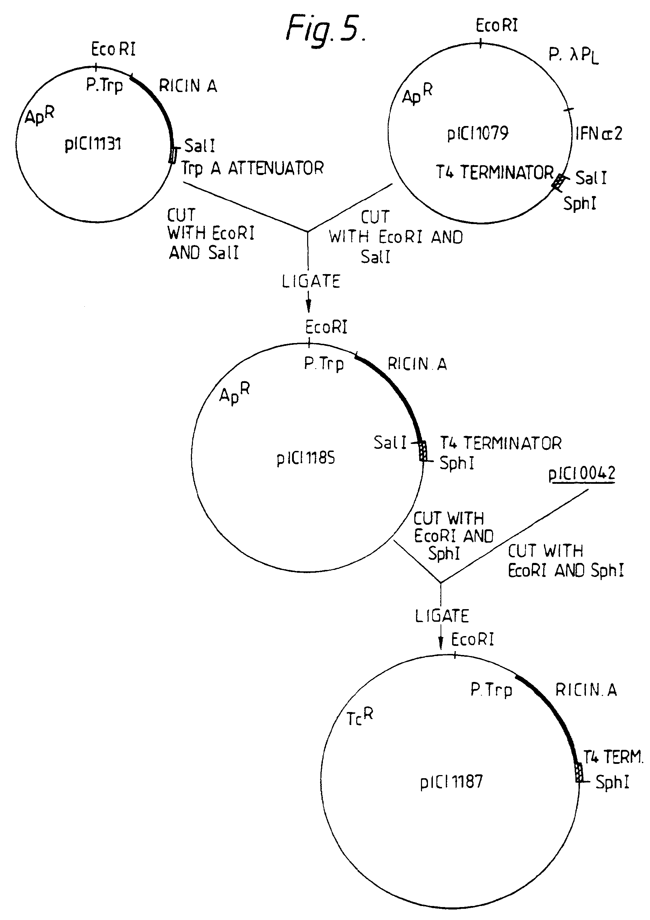

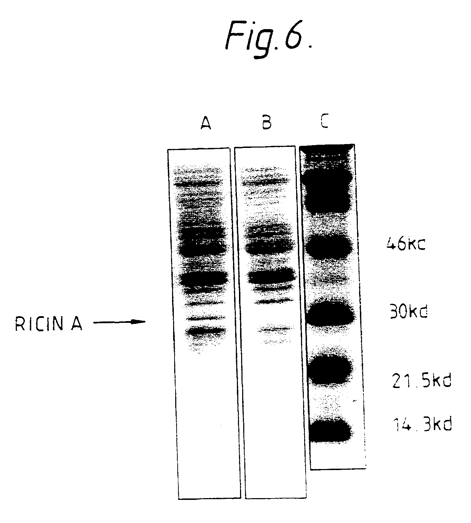

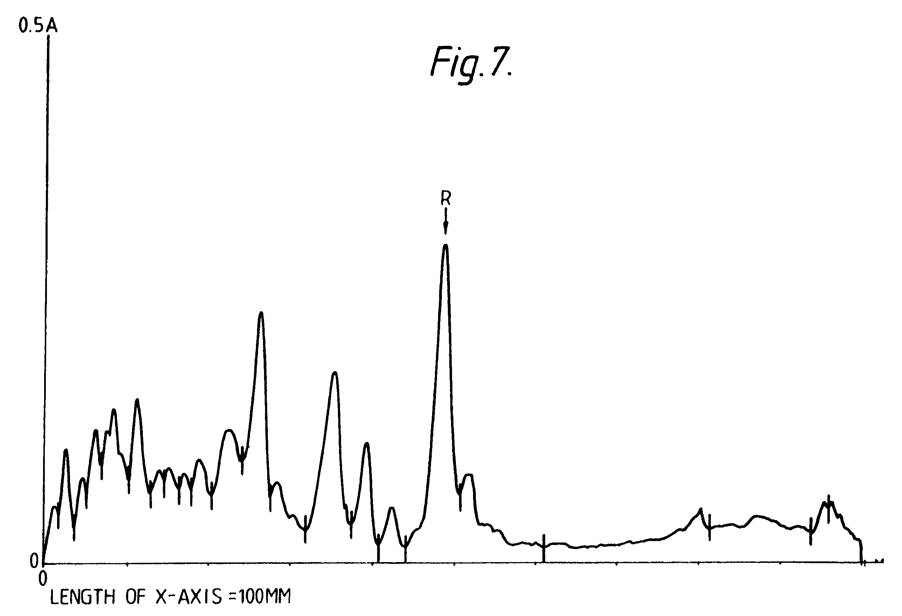

The construction of pICI 1102 is outlined in fig.5. Results of expression studies are shown in figs.6 and 7.

6.f) Western transfers and immunodetection of recombinant ricin A

Authenticity of recombinant ricin A-chain protein, initially observed by Coomassie blue staining of SDS-polyacrylamide gels, was confirmed by Western blotting. The protein bands were transferred to nitrocellulose filters and detected using a ricin A specific antibody followed by peroxidase labelled antiglobulins.

15% SDS-PAGE gels were run overnight at 8mA then equilibrated for at least 30 minutes in transfer buffer.

Protein bands on the gels were then transferred to nitrocellulose membranes (Hybond-C, Amersham) electrophoretically in a Bio-Rad Trans Blot apparatus at 70V for 3 hours. The filters could be stored, after drying, in sealed plastic bags at -20°C.

Ricin A.1 was a polyclonal antibody raised in rabbits against a synthetic peptide fragment of ricin A. Preliminary studies showed good affinity for ricin A but considerable cross-reactivity with many E.coli proteins. To overcome the high background caused by this cross-reactivity the antibody was pre-incubated with an E.coli lysate.

Thus, a 10ml L-broth overnight culture of E.coli strain DS410 was centrifuged at 4000 rpm for 10 minutes to pellet the cells. The pellet was resuspended in 5ml of bacterial buffer and sonicated at 4-6µ for 6 x 10 second bursts with 30 seconds cooling intervals on ice.

0.5ml of sonicate was then mixed with 0.5ml of ricin A.1 antiserum and incubated at room temperature for 90 minutes. Cell debris was spun down at 13000 rpm for 5 minutes and the supernate stored at -20°C.

The nitrocellulose filters from Western transfers were blocked by incubation overnight at room temperature in 5% BSA-PBS/Tween. (PBS Tween = 5ml Tween 20 per 1 litre of PBS).
Washed 3 x 3 minutes in PBS/Tween.
Incubated 2 hours (or overnight) at room temperature with a 1/4000 dilution of "blocked" Ricin A.1 antibody in 0.5% BSA-PBS/Tween.
Washed 3 x 3 minutes in PBS/Tween.
Incubated 1 hour with a 1/1000 dilution of goat anti rabbit antiserum in 0.5% BSA-PBS/Tween at room temperature.
Washed 3 x 3 minutes in PBS/Tween.
Incubated 1 hour with a 1/5000 dilution of rabbit peroxidase anti-peroxidase antiserum in 0.5% BSA/PBS/Tween at room temperature.
Washed 3 x 3 minutes in PBS/Tween.
Developed by immersion in a solution of 4-chloronaphthol (60mg) in 20ml methanol made to 120ml with PBS and containing 12µl hydrogen peroxide. The membrane was removed from the solution as soon as bands were visible, dried and photographed.

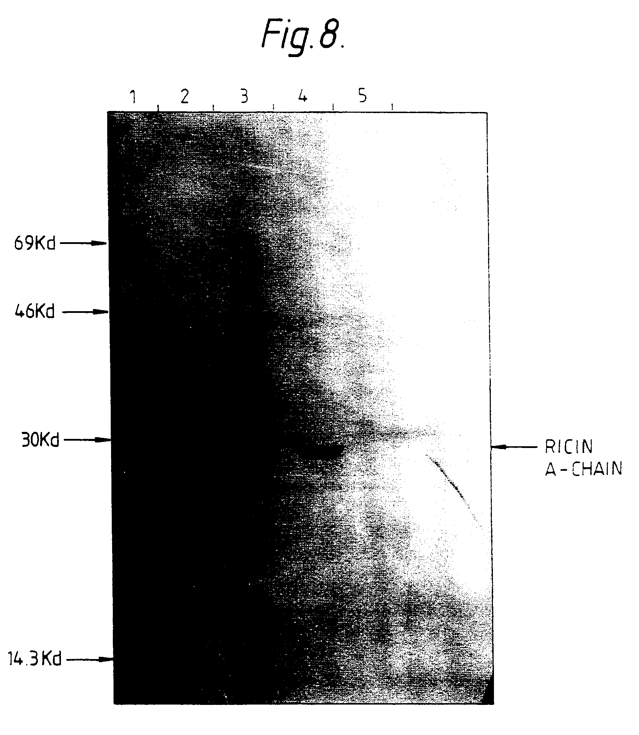

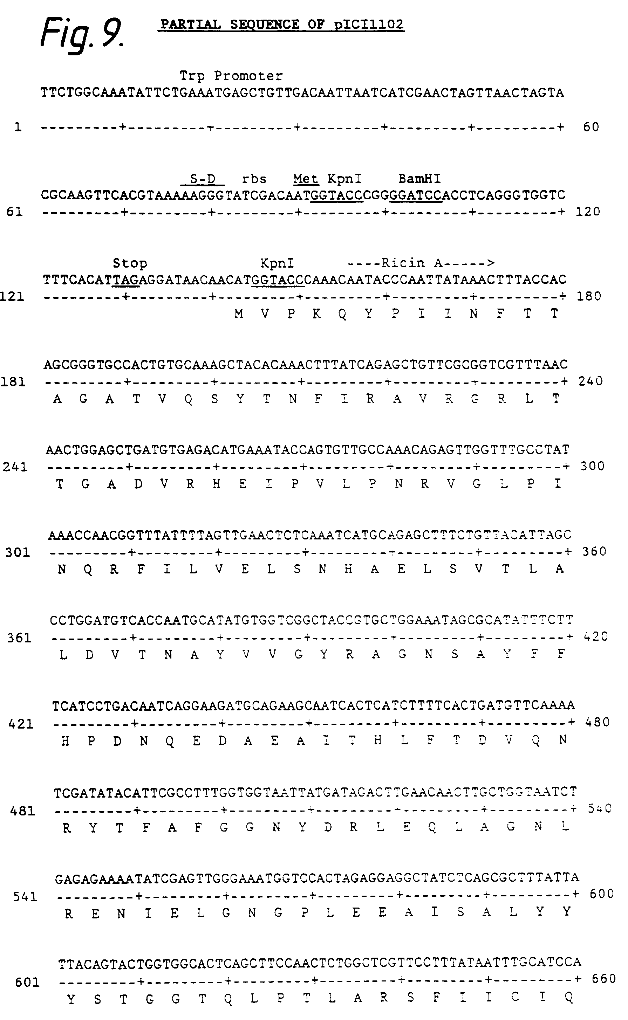

A typical Western blot analysis is shown in fig.8.

### 6.g) Biological assay for recombinant ricin A protein

The aim here was to establish conditions under which samples generated during the ricin A-chain purification from E.coli cells could be tested for biological activity in a cell-free in vitro protein synthesis assay.

Rabbit reticulocyte lysates were prepared according to the method of Allen and Schweet (J Biol Chem (1962), 237, 760-767). The assay demonstrates inhibition of protein synthesis in a cell-free system by a lack of incorporation of ¹⁴C-labelled leucine into newly synthesised protein.

### 6.g.i) The assay protocol

Stock solution: 1mM amino acid mix minus leucine. A solution containing all L-amino acids at 1mM except leucine (adjusted to pH7.4 with NaOH and stored at -70°C).
   Soln. A
      40mM Magnesium acetate
      2M Ammonium acetate
      0.2M Tris
      (pH 7.4 with HC1, stored 4°C)
   Soln. B
      ATP (Sigma A5394) 246mg/ml
      GTP (Sigma G8752) 24.4mg/ml
Assay mix: 1ml Amino acid mixture
   1ml Soln. A
   0.1ml Soln. B
   103mg Creatine phosphate
   1mg Creatine kinase
   510µl H₂O
   600µl (60µCi) L-¹⁴C-leucine (New England Nuclear, NEC-279E)
Reaction mix: Test sample 25µl
   Assay mix 12.5µl
   Rabbit reticulocyte lysate 25µl
   Blank solution was 2mg/ml BSA in PBS
   All assays were done in duplicate
   12.5µl of assay mix placed in sterile glass tubes
   25µl of BSA in PBS added to each of first four tubes for blanks
   25µl of test samples added to rest of tubes
   1ml 0.1M KOH added to first two tubes (background blank)
   Tubes equilibrated to 28°C in a water bath
   25µl of rabbit reticulocyte lysate (allowed to thaw from liquid nitrogen temperature) were added to each tube at 20 second intervals. When first tube had incubated for 12 minutes, 1ml 0.1M KOH was added to each tube again at 20 second intervals to allow all tubes to have 12 minutes incubation. Two drops of 20% hydrogen peroxide were added to each tube followed by 1ml of 20% TCA.
   Tubes were mixed and allowed to stand for at least 1 hour, or overnight, at 4°C. The precipitates were filtered on to 2.5 cm GFC discs, washed with 3 x 4 ml of 5% TCA, transferred to scintillation vials and 10ml scintillant (Ready-Solv. MP, Beckman) added. After 1 hour the vials were shaken and counted.

### 6.g.ii) Establishment of technique for use with E.coli lysates

10ml L-broth overnight cultures were grown at 37°C. 400µl aliquots were pelleted at 13000 rpm for 30 seconds and most of the supernate decanted.

The pellets were subjected to 2 rounds of rapid freezing in dry ice/EtOH followed by thawing at 37°C. 12µl of 25% sucrose in 50mM Tris HC1 pH8.0 were added followed by 4µl of a 10mg/ml solution of lysozyme.

After incubation on ice for 15 minutes, 8µl of 0.25M EDTA were added and incubation continued for 15 minutes. Lysis was brought about osmotically by diluting the samples to 400µl with water. This procedure produced viable cell counts of 80-100 per ml.

When a 25µl aliquot of this lysate was added into the assay reaction mix, the level of incorporation of ¹⁴C-leucine into newly synthesised protein was ∼10% of the blank without lysate. This was a similar level of inhibition to that produced by 8ng/ml ricin A. Dilutions of the E.coli lysate were then prepared and the assay repeated. The result clearly showed that a minimum 16-fold dilution was necessary to reduce the effect of the lysate to equal that of the blank.

In order to be as confident as possible that lysis of E.coli and E.coli lysates would not compromise ricin A toxicity, 2 control assays were performed. The first added plant-derived ricin A to a 16X diluted E.coli cell pellet so as to give a final concentration of 8ng/ml in the assay mix after cell lysis. Both these controls showed no deleterious affect from the lysates or the lysis procedure on the inhibitory action of ricin A.

These techniques were used to verify the synthesis of biologically active, recombinant ricin A from pICI 1102 and the clones described below.

### 6.h) DNA sequence analysis

Plasmid DNA sequencing was used to analyse pICI 1102. The protocol chosen was modified from Zagursky et al (Gene Analysis Techniques Vol 2, N° 5) and involves alkaline denaturation of double stranded plasmid DNA prior to primer annealing and sequencing by a standard procedure such as that provided in kit form by several suppliers, eg. Sequenase (United States Bioscience). By using an oligonucleotide to prime at the 3′ end of β-lactamase and several A-chain internal primers, sequencing both strands of the promoter and ricin A gene was possible.

The initial sequencing data revealed an unexpected result in that an additional KpnI fragment was present between the promoter and ricin A coding sequence, ie:

SEQ.ID NO 4

KpnI
------
5′ AAAAAGGGTATCGACATGGTACCCGGGGATCCACCTCAGGGTGG

KpnI
------
TCTTTCACATTAGAGGATAACAACATGGTACCCAAACAATAC 3′

The additional KpnI fragment has come from M13K19RA and contains restriction enzyme sites plus the part of the ricin leader sequence cloned from pUC8RA. The 5′ region of the ricin A chain contains the base changes induced during mutagenesis.

Study of this sequence reveals that the first translation initiation codon (ATG) is out of frame with that the ricin A coding region. Also, there is an in-frame termination codon (TAG) prior to the ricin A initiation codon and a putative Shine-Dalgarno sequence (AGGA) which could re-initiate translation from the second ATG.

Subsequent studies revealed that, surprisingly, this additional DNA fragment conferred a beneficial advantage with respect to the accumulation level of ricin A-chain in E.coli when compared to clones from which it had been excised.

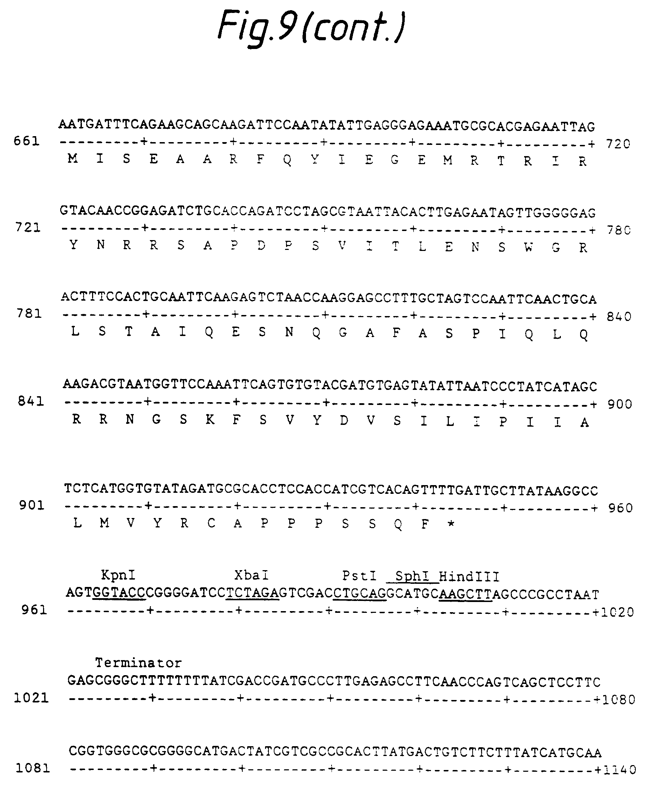

The complete DNA sequence of the ricin A gene contained in pICI 1102 is given in fig.9.

### 7. Generation of subsequent ricin A expressing clones

### 7.a) Mutation of Ricin-A clone pICI 1102 to allow subcloning

To subclone the two KpnI fragments from the fortuitously generated pICI 1120 in the correct orientation for ricin-A expression would be difficult. Consequently, we planned to alter the internal KpnI recognition site by a single base substitution (A to T). This would prevent KpnI cleavage at this site and allow the subcloning of a single KpnI fragment into the range of trp/RBS vectors. By substituting the adenine of the KpnI recognition site (GGTACC) with thymine (ie GGTTCC) the first residue of ricin-A is unaltered (GTA/GTT = Val).
ie: 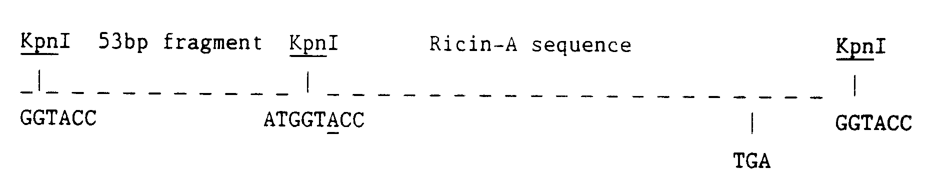
KpnI   53bp fragment   KpnI        Ricin-A sequence                    KpnI


GGTACC                ATGGTACC                                              GGTACC

                                                                  TGA
 Changed to:

53bp fragment          Ricin-A sequence

KpnI                                                    KpnI

GGTACC          ATGGTTCC                                GGTACC

                                        TGA

                not recognised by KpnI

The oligonucleotide synthesised to produce this change has the sequence: 5′ A T A A C A A C A T G G T T C C C A A A C A A T A C 3′ where the underlined base represents the mutational change.

We planned to clone the mutated ricin-A fragment into a range of trp expression vectors for comparative expression studies. Cloning into pICI 0020 provides a comparison with pICI 1102 to determine the effects on expression, if any, of the single base substitution.

### 7.b) Mutagenesis

The template for mutagenesis was MRA16 which is the M13 clone containing the two KpnI fragments present in pICI 1102. After mutagenesis, isolates carrying the desired mutations were identified by random sampling and DNA sequence determination over the region to which the mutagenic oligonucleotide binds specifically.

One mutated template was named MRA22. This was analysed further by DNA sequence determination of the entire ricin-A coding sequence to verify the absence of non-specific mutations.

### 7.c) Sub-cloning

The mutated, single-stranded DNAs were used to transform competent E.coli TG1 cells to produce single plaques. Individual plaques were then picked and replicative form (RF, double-stranded) DNA purified by banding on caesium chloride/ethidium bromide buoyant density gradients. The purified RF DNA was digested to completion with KpnI. Cloning was achieved by "shotgun" ligation of the digested RF DNA with the appropriate KpnI cut and phosphatased expression vector or by specific ligation of the ricin-A fragment after its purification from an agarose gel. Ligated DNA was transformed into E.coli TG1 or HB101.

Ricin-A containing clones were identified by hybridisation screening using a ³²P labelled ricin-A probe produced by random hexanucleotide priming of a KpnI fragment isolated from another ricin A containing clone (pICI 1121). Colonies showing positive hybridisation were screened further by restriction analysis of plasmid DNA using a KpnI single digest and an EcoRI/BglII double digest. KpnI identifies the size of the inserted fragment and EcoRI/BglII determines the orientation of the fragment.

Clones confirmed as having the ricin-A fragment in the correct orientation for expression were subjected to clone selection grows and analysis by SDS-PAGE followed by Coomassie staining and Western blotting of duplicate gels. The level of ricin A accumulation in these clones was equivalent to that detected from pICI 1102.

One isolate was selected and named pICI 1131.

### 7.d) Use of alternative transcription terminator element.

In these experiments, the trp promoter and ricin-A fragment from pICI 1131 was excised by digestion with the enzymes EcoRI and SalI. The latter enzyme cleaves between the 3′ terminus of the ricin-A coding sequence and the trpA transcription terminator. The resulting fragment was excised from an agarose gel (2% NuSieve GTG Agarose, FMC Bioproducts) and purified by phenol and chloroform. extractions followed by ethanol precipitation. The purified fragment was ligated with pICI 1079 cut with EcoRI and SalI. This latter plasmid contains the T₄ terminator between unique SalI and SphI sites.

Ligated DNA was used to transform competent E.coli HB101 (BRL) and hybridisation screening used to detect the presence of ricin-A DNA as in previous experiments. Positively hybridising clones were chosen for plasmid DNA preparation followed by restriction analysis with EcoRI and SalI together to show the presence of an appropriately sized fragment.

One isolate with the correct construction was identified and named pICI 1185.

### 7.e) The use of alternative plasmid background.

Plasmid DNA was prepared from pICI 1185 and digested with EcoRI and SphI together to excise an expression cassette containing the trp promoter/RBS1/ricin-A (MRA22) fragment/T₄ terminator. This fragment was isolated by the method outlined in 6.d and ligated with pICI 0042 cut with EcoRI and SphI.

Ligated DNA was used to transform E.coli HB101. HB101 transformations were plated on L agar + tetracycline, incubated at 37°C overnight, and colonies screened by hybridisation with a ³²P labelled ricin-A DNA probe.

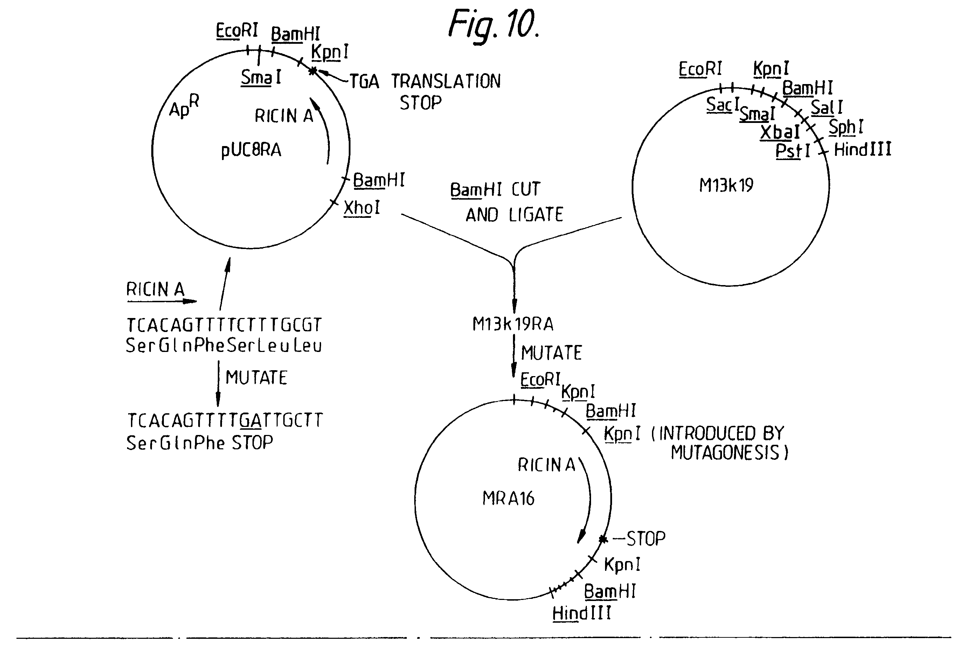

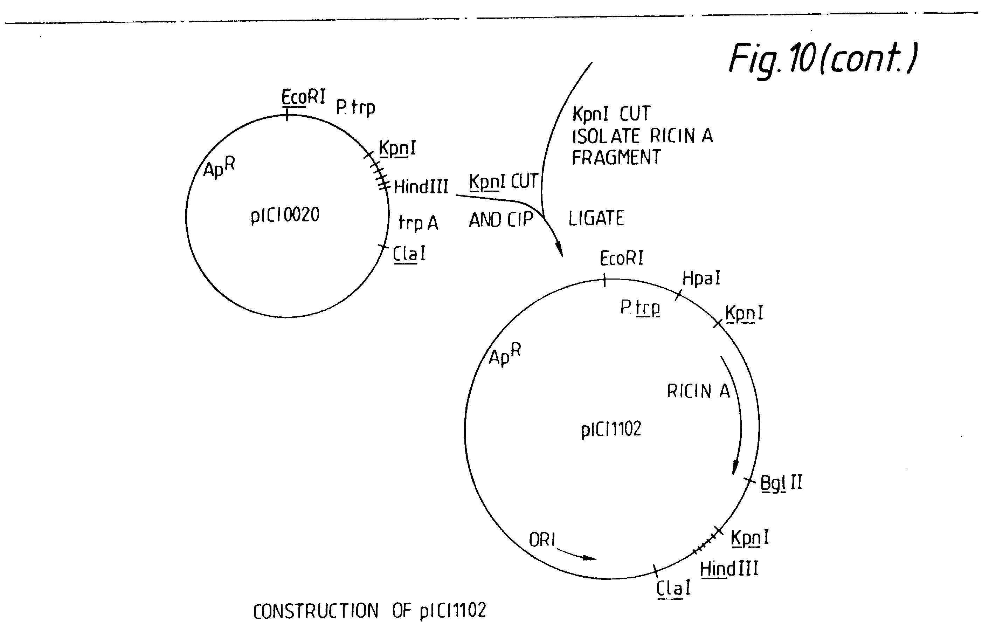

In both cases, positively identified colonies were confirmed by restriction analysis of plasmid DNA using EcoRI/SphI and EcoRI/BglII digests. Three isolates were identified, one of which (pICI 1187) was used in the fermentations described above. Figure 10 outlines the construction of pICI 1187.

SEQUENCE LISTING

(1) GENERAL INFORMATION

(i) APPLICANT: Imperial Chemical Industries PLC

(ii) TITLE OF INVENTION: FERMENTATION PROCESSES

(iii) NUMBER OF SEQUENCES: 4

(iv) CORRESPONDENCE ADDRESS:

(A) ADDRESSEE: Legal Department: Patents

(B) STREET: Bessemer Road

(C) CITY: Welwyn Garden City

(D) STATE: Hertfordshire

(E) COUNTRY: United Kingdom

(F) ZIP: GB-AL7 1HD

(vi) CURRENT APPLICATION DATA:

(A) APPLICATION NUMBER:

(B) FILING DATE:

The following is a list of the sequences referred to in the application.
The sequences are written in the conventional 5′ to 3′ sense.

SEQ ID No 1
SEQUENCE LENGTH:  21 bases
SEQUENCE TYPE:    Nucleotide
STRANDEDNESS:     Single
TOPOLOGY:         Linear

GATAACAACA TATTCCCCAA A                                    21

SEQ ID No 2
SEQUENCE LENGTH:  21 bases
SEQUENCE TYPE:    Nucleotide
STRANDEDNESS:     Single
TOPOLOGY:         Linear

GATAACAACA TGGTACCCAA A                                    21

SEQ ID No 3
SEQUENCE LENGTH:  21 bases
SEQUENCE TYPE:    Nucleotide
STRANDEDNESS:     Single
TOPOLOGY:         Linear

AACAACATGG TACCCAAACA A                                    21

SEQ ID No 4
SEQUENCE LENGTH:  86 bases
SEQUENCE TYPE:    Nucleotide
STRANDEDNESS:     Single
TOPOLOGY:         Linear

AAAAAGGGTA TCGACATGGT ACCCGGGGAT CCACCTCAGG GTGGTCTTTC ACATTAGAGG 60
ATAACAACAT GGTACCCAAA CAATAC 86

## Claims

1. A process for preparing a polypeptide, which process comprises cultivating a host capable of expressing said polypeptide in a growth medium and cultivating said host for an initial period at a first pH value which favours growth of the host so that polypeptide is produced;
adjusting the pH to a second value which favours accumulation of soluble polypeptide and cultivating the host for a further period at said second pH value;
and optionally reducing the temperature of the growth medium during the terminal portion of the cultivation;
such that soluble polypeptide may be obtained.

2. A process as claimed in claim 1 wherein the pH is adjusted by lowering its value from a first value to a second value.

3. A process as claimed in claim 1 or 2 wherein the pH is adjusted from a value of about 6.7 to a lower value which is greater than 5.5 and less than 6.7.

4. A process as claimed in claim 1, 2 or 3 wherein the process is a fermentation of the fed-batch type and wherein the pH is adjusted before fed-batch conditions are attained.

5. A process as claimed in any one of the preceding claims wherein the host comprises E.coli and the polypeptide comprises ricin A or an analogue thereof.

6. A process as claimed in claim 5 wherein the host comprises E.coli DS410.

7. A process as claimed in claim 1 in which a host capable of expressing ricin A, or an analogue thereof, is cultivated in a growth medium and a supplement which includes yeast extract is added to the growth medium during the cultivation.

8. A process as claimed in any one of the preceding claims wherein the temperature is decreased from a first value of about 37°C to a value below about 25°C.

9. A process as claimed in any one of the preceding claims wherein the temperature is decreased from a first value of about 37°C to a value below about 25°C.

10. A process for preparing ricin A which comprises a fermentation of the fed-batch type in which an E.coli host which is capable of expressing ricin A is cultivated in a growth medium at a pH of about 6.7, the pH is reduced to a value which is greater than 5.5 and less than 6.7 and cultivated for a further period, and optionally reducing the temperature of the growth medium during the terminal portion of the cultivating and harvesting the ricin A during said terminal portion.

## Patentansprüche

1. Verfahren zur Herstellung eines Polypeptids, wobei das Verfahren umfaßt: Kultivieren eines zur Expression des Polypeptids fähigen Wirtes in einem Wachstumsmedium und Kultivieren des Wirtes für einen Anfangszeitraum bei einem ersten pH-Wert, der das Wachstum des Wirtes begünstigt, so daß Polypeptid erzeugt wird; Einstellen des pH auf einen zweiten Wert, der die Anreicherung von löslichem Polypeptid begünstigt, und Kultivieren des Wirtes für einen weiteren Zeitraum bei dem zweiten pH-Wert; und gegebenenfalls Reduzieren der Temperatur des Wachstumsmediums während des Endbereiches der Kultivierung; so daß lösliches Polypeptid erhalten werden kann.

2. Verfahren gemäß Anspruch 1, worin der pH eingestellt wird, indem sein Wert von einem ersten Wert auf einen zweiten Wert abgesenkt wird.

3. Verfahren gemäß Anspruch 1 oder 2, worin der pH von einem Wert von ca. 6,7 auf niedrigeren Wert eingestellt wird, der größer als 5,5 und kleiner als 6,7 ist.

4. Verfahren gemäß Anspruch 1, 2 oder 3, worin das Verfahren eine Fermentation des Fed-Batch-Typs ist und worin der pH eingestellt wird, bevor Fed-Batch-Bedingungen erreicht sind.

5. Verfahren gemäß einem der vorhergehenden Ansprüche, worin der Wirt E. coli umfaßt und das Polypeptid Ricin A oder ein Analogon davon umfaßt.

6. Verfahren gemäß Anspruch 5, worin der Wirt E. coli DS410 umfaßt.

7. Verfahren gemäß Anspruch 1, worin ein zur Expression von Ricin A oder einem Analogon davon fähiger Wirt in einem Wachstumsmedium kultiviert wird und ein Ergänzungsmittel, das Hefeextrakt einschließt, zum Wachstumsmedium während der Kultivierung hinzugegeben wird.

8. Verfahren gemäß einem der vorhergehenden Ansprüche, worin die Temperatur von einem ersten Wert von ca. 37°C auf einen Wert unterhalb ca. 25°C verringert wird.

9. Verfahren gemäß einem der vorhergehenden Ansprüche, worin die Temperatur von einem ersten Wert von ca. 37°C auf einen Wert unterhalb ca. 25°C verringert wird.

10. Verfahren zur Herstellung von Ricin A, welches eine Fermentation des Fed-Batch-Typs umfaßt, worin ein E. coli-Wirt, der zu Expression von Ricin A fähig ist, in einem Wachstumsmedium bei einem pH von 6,7 kultiviert wird, der pH auf einen Wert, der größer als 5,5 und niedriger als 6,7 ist, reduziert wird und für einen weiteren Zeitraum kultiviert wird und gegebenenfalls die Temperatur des Wachstumsmediums während des Endbereiches der Kultivierung reduziert wird und das Ricin A während des Endbereiches geerntet wird.

## Revendications

1. Procédé pour la préparation d'un polypeptide, procédé qui comprend la culture d'un hôte capable d'exprimer ledit polypeptide dans un milieu de croissance et la culture dudit hôte pendant une période initiale à une première valeur de pH qui favorise la croissance de l'hôte de telle sorte que le polypeptide soit produit ;
l'ajustement du pH à une seconde valeur qui favorise l'accumulation du polypeptide soluble et la culture de l'hôte pendant une période supplémentaire à ladite seconde valeur de pH ;
et, facultativement, l'abaissement de la température du milieu de croissance au cours de la partie terminale de la culture ;
de telle sorte que le polypeptide soluble puisse être obtenu.

2. Procédé suivant la revendication 1, dans lequel le pH est ajusté en abaissant sa valeur d'une première valeur à une seconde valeur.

3. Procédé suivant la revendication 1 ou 2, dans lequel le pH est ajusté d'une valeur d'environ 6,7 à une valeur plus basse qui est supérieure à 5,5 et inférieure à 6,7.

4. Procédé suivant la revendication 1, 2 ou 3, qui consiste en une fermentation de type à alimentation discontinue et dans lequel le pH est ajusté avant que ne soient atteintes les conditions d'alimentation discontinue.

5. Procédé suivant l'une quelconque des revendications précédentes, dans lequel l'hôte comprend E. coli et le polypeptide comprend la ricine A ou un de ses analogues.

6. Procédé suivant la revendication 5, dans lequel l'hôte comprend E. coli DS410.

7. Procédé suivant la revendication 1, dans lequel un hôte capable d'exprimer la ricine A, ou un de ses analogues, est cultivé dans un milieu de croissance et un supplément qui comprend un extrait de levure est ajouté au milieu de croissance au cours de la culture.

8. Procédé suivant l'une quelconque des revendications précédentes, dans lequel la température est abaissée d'une première valeur d'environ 37°C à une valeur inférieure à environ 25°C.

9. Procédé suivant l'une quelconque des revendications précédentes, dans lequel la température est abaissée d'une première valeur d'environ 37°C à une valeur inférieure à environ 25°C.

10. Procédé pour la préparation de ricine A, qui comprend une fermentation de type à alimentation discontinue dans laquelle un hâte consistant en E. coli, qui est capable d'exprimer la ricine A, est cultivé dans un milieu de croissance à un pH d'environ 6,7, le pH est abaissé à une valeur qui est supérieure à 5,5 et inférieure à 6,7 et la culture est effectuée pendant une période supplémentaire et, facultativement, la température du milieu de croissance est abaissée au cours de la partie terminale de la culture et la ricine A est recueillie au cours de ladite partie terminale.
